# EUROPEAN PATENT SPECIFICATION

(11) **EP 4 319 621 B1**
(45) Date of publication and mention of the grant of the patent: **05.08.2026**
(21) Application number: 22729397.4
(22) Date of filing: 20.05.2022
(51) Int. Cl.: A61B 5/00, A61B 5/0215, A61F 2/24, A61M 27/00

(54) **SHUNT BARREL SENSOR IMPLANT ANCHORING**
SHUNT-BARREL-SENSORIMPLANTATVERANKERUNG
ANCRAGE D'IMPLANT DE CAPTEUR DE CYLINDRE DE DÉRIVATION

(30) Priority: 21.05.2021 US 202163191534 P; 21.07.2021 US 202163224286 P; 23.07.2021 US 202163225039 P; 26.07.2021 US 202163225689 P; 19.08.2021 US 202163235038 P
(43) Date of publication of application: 14.02.2024
(73) Proprietor: Edwards Lifesciences Corporation, Irvine, CA 92614-5688 (US)
(72) Inventor: MCCONNELL, Steven, Anaheim, CA 92808 (US); VALDEZ, Michael, G., Irvine, CA 92614 (US); CHANG, Arvin, T., Irvine, CA 92614 (US); AMEFIA, Kokou, Anani, Aliso Viejo, CA 92656 (US); RABBAH, Jean-Pierre, Michel, Irvine, CA 92614 (US); MAHMOUDI, Rani, Abdullah, Irvine, CA 92614 (US); HINZMAN, Julie, Ann, Irvine, CA 92606 (US); POOL, Scott, Louis, Irvine, CA 92614 (US)
(74) Representative: Venner, Julia Ann
(86) International application number: PCT/US2022/030206
(87) International publication number: WO 2022/246166

(56) References cited:
- WO-A1-2020/123338
- WO-A1-2020/163112
- WO-A1-2021/086707
- US-A1- 2008 071 178
- US-A1- 2016 045 165
- YEH CHAO-CHI ET AL: "Fabrication of a flexible wireless pressure sensor for intravascular blood pressure monitoring", MICROELECTRONIC ENGINEERING, vol. 213, 11 April 2019 (2019-04-11), pages 55 - 61, XP085679189, ISSN: 0167-9317, DOI: 10.1016/J.MEE.2019.04.009

## Description

### RELATED APPLICATIONS

This application claims priority based on United States Provisional Patent Application Serial No. 63/191,534, filed May 21, 2021 and entitled IMPLANT-COUPLED SENSORS; United States Provisional Patent Application Serial No. 63/224,286, filed July 21, 2021 and entitled IMPLANT-ADJACENT SENSOR ANCHORING; United States Provisional Patent Application Serial No. 63/225,039, filed July 23, 2021 and entitled SHUNT BARREL SENSOR IMPLANT ANCHORING; United States Provisional Patent Application Serial No. 63/225,689, filed July 26, 2021 and entitled EMBEDDED SENSOR IMPLANT DEVICES; and United States Provisional Patent Application Serial No. 63/235,038, filed August 19, 2021 and entitled SENSOR IMPLANT DEVICE ANCHORING.

### BACKGROUND

### Field

The present disclosure generally relates to the field of medical implant devices.

### Description of Related Art

Various medical procedures involve the implantation of medical implant devices within the anatomy of the heart. Certain physiological parameters associated with such anatomy, such as fluid pressure, can have an impact on patient health prospects.

WO 2020/123338 describes a sensor implant device including a shunt structure comprising a flow path conduit and a plurality of arms configured to secure the shunt structure to a tissue wall, and a pressure sensor device attached to one of the plurality of arms of the shunt structure. US 2016/0045165 describes prosthetic heart devices which may be implanted into the heart with a sensor coupled to the device, the sensor being configured to measure physiological data, such as blood pressure, in the heart. The sensors may include a body with different configurations for attaching to the implantable device, such as apertures for sutures or fingers for connecting to structures of the implantable device. WO 2021/086707 describes a sensor-retention structure including a sensor-support strut and means for securing sensor device to the sensor-support strut.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

Described herein are one or more methods (not forming part of the presently claimed invention) and/or devices to facilitate monitoring of physiological parameter(s) associated with certain chambers and/or vessels of the heart, such as the left atrium, using one or more sensor implant devices.

For purposes of summarizing the disclosure, certain aspects, advantages and novel features have been described. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the inventions. In addition, various features of different disclosed examples can be combined to form additional examples, which are part of this disclosure. Throughout the drawings, reference numbers may be reused to indicate correspondence between reference elements.
Figure 1 illustrates an example representation of a human heart in accordance with one or more examples.
Figure 2 illustrates example pressure waveforms associated with various chambers and vessels of the heart according to one or more examples.
Figure 3 illustrates a graph showing left atrial pressure ranges.
Figure 4 is a block diagram representing an implant device in accordance with one or more examples.
Figure 5 is a block diagram representing a system for monitoring one or more physiological parameters associated with a patient according to one or more examples.
Figure 6 illustrates an example sensor assembly/device that can be a component of a sensor implant device, in accordance with one or more examples.
Figure 7 illustrates an example shunt/anchor structure which may be configured for attachment to one or more sensor devices, in accordance with one or more examples.
Figure 8 shows a shunt implant/anchor device/structure implanted in an atrial septum in accordance with one or more examples.
Figure 9 shows a shunt device/structure implanted in a tissue wall between the coronary sinus and the left atrium.
Figures 10A and 10B illustrate example sensor implant devices in accordance with one or more examples of the present disclosure.
Figures 11A and 11B illustrate example sensor implant devices in accordance with one or more examples of the present disclosure.
Figures 12A and 12B illustrate a sensor implant device comprising a sensor coupled to and/or extending from a shunt body comprising one or more wire loops, in accordance with one or more embodiments of the presently claimed invention.
Figures 13A-13D illustrate another sensor implant device comprising a sensor coupled to a coil shunt body configured to secure the sensor at a desired position within a heart, in accordance with one or more embodiments of the presently claimed invention.
Figure 14 provides a side view of an example sensor implant device comprising a sensor dock configured to couple to and/or otherwise mate with one or more sensors in accordance with one or more embodiments of the presently claimed invention.
Figure 15 provides a side view of an example sensor implant device comprising a sensor dock configured to couple to and/or otherwise mate with one or more sensors, in accordance with one or more embodiments of the presently claimed invention.
Figure 16 provides an overhead view of an example sensor implant device comprising a sensor dock configured to couple to and/or otherwise mate with one or more sensors, in accordance with one or more embodiments of the presently claimed invention.
Figure 17 illustrates an example sensor implant device with a shunt implant in accordance with one or more embodiments of the presently claimed invention.
Figures 18A and 18B illustrate a sensor implant device comprising a sensor coupled to one or more self-expanding anchoring arms via one or more tethers, in accordance with one or more embodiments of the presently claimed invention.
Figures 19A and 19B illustrate a sensor implant device comprising a sensor coupled to a first implant via one or more tethers, in accordance with one or more examples.
Figures 20A and 20B illustrate a sensor implant device comprising a sensor coupled to a first implant via one or more coupling arms, in accordance with one or more examples.
Figures 21A-21C illustrate another sensor implant device comprising a sensor coupled to a barrel portion of the sensor implant device, in accordance with one or more embodiments of the presently claimed invention.
Figure 22 provides a flowchart illustrating steps of a process for delivering the one or more sensor implant devices and/or shunt devices described herein, in accordance with one or more examples.

### DETAILED DESCRIPTION

The headings provided herein are for convenience only and do not necessarily affect the scope or meaning of the claimed invention.

Although certain preferred examples and examples are disclosed below, inventive subject matter extends beyond the specifically disclosed examples to other alternative examples and/or uses and to modifications and equivalents thereof. Thus, the scope of the claims that may arise herefrom is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain reference numbers are re-used across different figures of the figure set of the present disclosure as a matter of convenience for devices, components, systems, features, and/or modules having features that may be similar in one or more respects. However, with respect to any of the examples disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

The present disclosure relates to systems, devices, and methods for monitoring of one or more physiological parameters of a patient (e.g., blood pressure) using sensor-integrated cardiac shunts and/or other medical implant devices. **In** some implementations, the present disclosure relates to cardiac shunts and/or other cardiac implant devices that incorporate or are associated with pressure sensors or other sensor devices. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly. Certain examples are disclosed herein in the context of cardiac implant devices. However, although certain principles disclosed herein are particularly applicable to the anatomy of the heart, it should be understood that sensor implant devices in accordance with the present disclosure may be implanted in, or configured for implantation in, any suitable or desirable anatomy.

While the various sensor devices described herein may be integrated with the various medical implant devices described herein, the sensor devices may be separate devices from the medical implant devices. For example, a sensor device may form a breakable and/or releasable connection with a medical implant device. Moreover, sensor devices described herein may be configured to be delivered separately (e.g., before and/or after) medical implant devices within a heart of a patient. For example, a sensor device may not be attached to a medical implant device during delivery processes (e.g., during delivery through a catheter) of the sensor device and/or medical implant device but may be attached/coupled to the medical implant device following delivery (e.g., following removal from a catheter) to a desired location within the heart. Example delivery locations can include the left atrium, the left atrial appendage, the pulmonary vein, the coronary sinus, and/or the various tissue walls associated with these locations.

In some examples, a catheter and/or guidewire used for delivering a sensor device may also be used for delivering a medical implant device. For example, the catheter and/or guidewire may remain within the body following delivery of the sensor device and/or medical implant device for delivery of the remaining device(s).

Some sensor devices described herein may be configured to be delivered prior to delivery of the medical implant devices described herein. This may advantageously simplify delivery of the sensor devices and/or medical implant devices and/or may provide for simple imaging the sensor devices and/or medical implant devices. The sensor devices may be adjusted as necessary to maximize measurements of the sensor devices prior to delivery of the medical implant devices. Moreover, delivery of the medical implant devices may be delayed and/or aborted as needed following delivery of the sensor devices.

Some sensor devices described herein may be configured to be delivered after delivery of the medical implant devices described herein. This may advantageously simplify delivery of the sensor devices and/or medical implant devices and/or may provide for simple imaging the sensor devices and/or medical implant devices. The sensor devices may be adjusted as necessary to maximize measurements of the sensor devices. Moreover, the sensor device may be effectively secured to the medical implant devices with minimal risk of dislodgement of the sensor devices.

### Cardiac Physiology

The anatomy of the heart is described below to assist in the understanding of certain inventive concepts disclosed herein. In humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

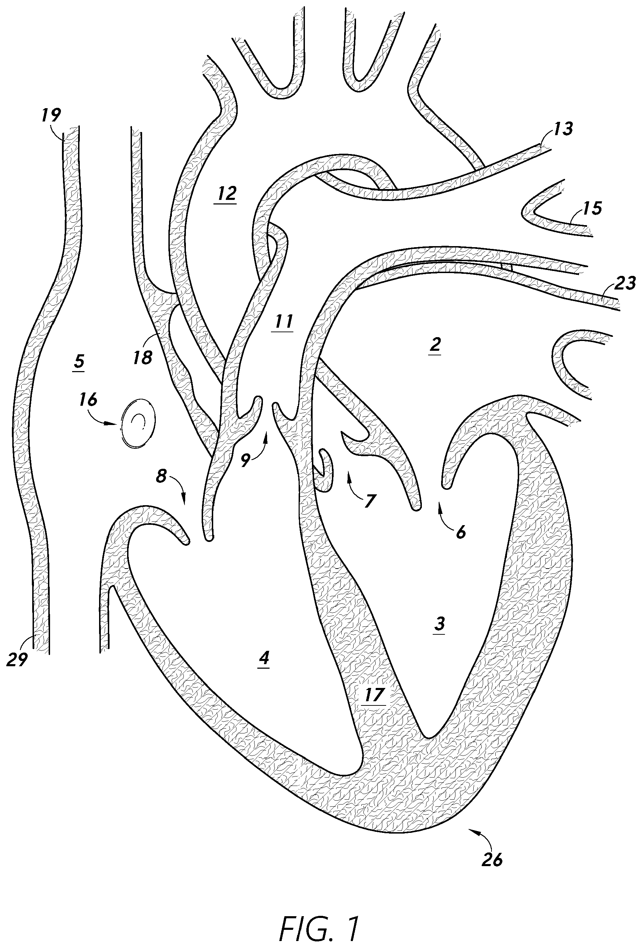

Figure 1 illustrates an example representation of a heart 1 having various features relevant to certain examples of the present inventive disclosure. The heart 1 includes four chambers, namely the left atrium 2, the left ventricle 3, the right ventricle 4, and the right atrium 5. In terms of blood flow, blood generally flows from the right ventricle 4 into the pulmonary artery 11 via the pulmonary valve 9, which separates the right ventricle 4 from the pulmonary artery 11 and is configured to open during systole so that blood may be pumped toward the lungs and close during diastole to prevent blood from leaking back into the heart from the pulmonary artery 11. The pulmonary artery 11 carries deoxygenated blood from the right side of the heart to the lungs. The pulmonary artery 11 includes a pulmonary trunk and left 15 and right 13 pulmonary arteries that branch off of the pulmonary trunk, as shown. The pulmonary veins 23 carry blood from the lungs to the left atrium 2.

In addition to the pulmonary valve 9, the heart 1 includes three additional valves for aiding the circulation of blood therein, including the tricuspid valve 8, the aortic valve 7, and the mitral valve 6. The tricuspid valve 8 separates the right atrium 5 from the right ventricle 4. The tricuspid valve 8 generally has three cusps or leaflets and may generally close during ventricular contraction (i.e., systole) and open during ventricular expansion (i.e., diastole). The mitral valve 6 generally has two cusps/leaflets and separates the left atrium 2 from the left ventricle 3. The mitral valve 6 is configured to open during diastole so that blood in the left atrium 2 can flow into the left ventricle 3, and, when functioning properly, closes during systole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

The heart valves may generally comprise a relatively dense fibrous ring, referred to herein as the annulus, as well as a plurality of leaflets or cusps attached to the annulus. Generally, the size of the leaflets or cusps may be such that when the heart contracts the resulting increased blood pressure produced within the corresponding heart chamber forces the leaflets at least partially open to allow flow from the heart chamber. As the pressure in the heart chamber subsides, the pressure in the subsequent chamber or blood vessel may become dominant and press back against the leaflets. As a result, the leaflets/cusps come in apposition to each other, thereby closing the flow passage. Dysfunction of a heart valve and/or associated leaflets (e.g., pulmonary valve dysfunction) can result in valve leakage and/or other health complications.

The atrioventricular (i.e., mitral and tricuspid) heart valves may further comprise a collection of chordae tendineae and papillary muscles (not shown) for securing the leaflets of the respective valves to promote and/or facilitate proper coaptation of the valve leaflets and prevent prolapse thereof. The papillary muscles, for example, may generally comprise finger-like projections from the ventricle wall. The valve leaflets are connected to the papillary muscles by the chordae tendineae. A wall of muscle, referred to as the septum, separates the left-side chambers from the right-side chambers. In particular, an atrial septum wall portion 18 (referred to herein as the "atrial septum," "interatrial septum," or "septum") separates the left atrium 2 from the right atrium 5, whereas a ventricular septum wall portion 17 (referred to herein as the "ventricular septum," "interventricular septum," or "septum") separates the left ventricle 3 from the right ventricle 4. The inferior tip 26 of the heart 1 is referred to as the apex and is generally located on or near the midclavicular line, in the fifth intercostal space.

The coronary sinus 16 comprises a collection of veins joined together to form a large vessel that collects blood from the heart muscle (myocardium). The ostium of the coronary sinus, which can be guarded at least in part by a Thebesian valve in some patients, is open to the right atrium 5, as shown. The coronary sinus runs along a posterior aspect of the left atrium 2 and delivers less-oxygenated blood to the right atrium 5. The coronary sinus generally runs transversely in the left atrioventricular groove on the posterior side of the heart.

Any of several access pathways in the heart 1 may be utilized for maneuvering guidewires and catheters in and around the heart 1 to deploy implants and/or devices of the present application. For instance, access may be from above via either the subclavian vein or jugular vein into the superior vena cava (SVC) 19, right atrium 5, and from there into the coronary sinus 16. Alternatively, the access path may start in the femoral vein and through the inferior vena cava (IVC) 14 into the heart 1. Other access routes may also be used, and each can utilize a percutaneous incision through which the guidewire and catheter are inserted into the vasculature, normally through a sealed introducer, and from there the physician can control the distal ends of the devices from outside the body.

### Health Conditions Associated with Cardiac Pressure and Other Parameters

As referenced above, certain physiological conditions or parameters associated with the cardiac anatomy can impact the health of a patient. For example, congestive heart failure is a condition associated with the relatively slow movement of blood through the heart and/or body, which causes the fluid pressure in one or more chambers of the heart to increase. As a result, the heart does not pump sufficient oxygen to meet the body's needs. The various chambers of the heart may respond to pressure increases by stretching to hold more blood to pump through the body or by becoming relatively stiff and/or thickened. The walls of the heart can eventually weaken and become unable to pump as efficiently. In some cases, the kidneys may respond to cardiac inefficiency by causing the body to retain fluid. Fluid build-up in arms, legs, ankles, feet, lungs, and/or other organs can cause the body to become congested, which is referred to as congestive heart failure. Acute decompensated congestive heart failure is a leading cause of morbidity and mortality, and therefore treatment and/or prevention of congestive heart failure is a significant concern in medical care.

The treatment and/or prevention of heart failure (e.g., congestive heart failure) can advantageously involve the monitoring of pressure in one or more chambers or regions of the heart or other anatomy. As described above, pressure buildup in one or more chambers or areas of the heart can be associated with congestive heart failure. Without direct or indirect monitoring of cardiac pressure, it can be difficult to infer, determine, or predict the presence or occurrence of congestive heart failure. For example, treatments or approaches not involving direct or indirect pressure monitoring may involve measuring or observing other present physiological conditions of the patient, such as measuring body weight, thoracic impedance, right heart catheterization, or the like. **In** some solutions, pulmonary capillary wedge pressure can be measured as a surrogate of left atrial pressure. For example, a pressure sensor may be disposed or implanted in the pulmonary artery, and readings associated therewith may be used as a surrogate for left atrial pressure. However, with respect to catheter-based pressure measurement in the pulmonary artery or certain other chambers or regions of the heart, use of invasive catheters may be required to maintain such pressure sensors, which may be uncomfortable or difficult to implement. Furthermore, certain lung-related conditions may affect pressure readings in the pulmonary artery, such that the correlation between pulmonary artery pressure and left atrial pressure may be undesirably attenuated. As an alternative to pulmonary artery pressure measurement, pressure measurements in the right ventricle outflow tract may relate to left atrial pressure as well. However, the correlation between such pressure readings and left atrial pressure may not be sufficiently strong to be utilized in congestive heart failure diagnostics, prevention, and/or treatment.

Additional solutions may be implemented for deriving or inferring left atrial pressure. For example, the E/A ratio, which is a marker of the function of the left ventricle of the heart representing the ratio of peak velocity blood flow from gravity in early diastole (the E wave) to peak velocity flow in late diastole caused by atrial contraction (the A wave), can be used as a surrogate for measuring left atrial pressure. The E/A ratio may be determined using echocardiography or other imaging technology; generally, abnormalities in the E/A ratio may suggest that the left ventricle cannot fill with blood properly in the period between contractions, which may lead to symptoms of heart failure, as explained above. However, E/A ratio determination generally does not provide absolute pressure measurement values.

Various methods for identifying and/or treating congestive heart failure involve the observation of worsening congestive heart failure symptoms and/or changes in body weight. However, such signs may appear relatively late and/or be relatively unreliable. For example, daily bodyweight measurements may vary significantly (e.g., up to 9% or more) and may be unreliable in signaling heart-related complications. Furthermore, treatments guided by monitoring signs, symptoms, weight, and/or other biomarkers have not been shown to substantially improve clinical outcomes. **In** addition, for patients that have been discharged, such treatments may necessitate remote telemedicine systems.

The present disclosure provides systems, devices, and methods for guiding the administration of medication relating to the treatment of congestive heart failure at least in part by directly monitoring pressure in the left atrium, or other chamber or vessel for which pressure measurements are indicative of left atrial pressure and/or pressure levels in one or more other vessels/chambers, such as for congestive heart failure patients in order to reduce hospital readmissions, morbidity, and/or otherwise improve the health prospects of the patient.

### Cardiac Pressure Monitoring

Cardiac pressure monitoring in accordance with examples of the present disclosure may provide a proactive intervention mechanism for preventing or treating congestive heart failure and/or other physiological conditions. Generally, increases in ventricular filling pressures associated with diastolic and/or systolic heart failure can occur prior to the occurrence of symptoms that lead to hospitalization. For example, cardiac pressure indicators may present weeks prior to hospitalization with respect to some patients. Therefore, pressure monitoring systems in accordance with examples of the present disclosure may advantageously be implemented to reduce instances of hospitalization by guiding the appropriate or desired titration and/or administration of medications before the onset of heart failure.

Dyspnea represents a cardiac pressure indicator characterized by shortness of breath or the feeling that one cannot breathe well enough. Dyspnea may result from elevated atrial pressure, which may cause fluid buildup in the lungs from pressure back-up. Pathological dyspnea can result from congestive heart failure. However, a significant amount of time may elapse between the time of initial pressure elevation and the onset of dyspnea, and therefore symptoms of dyspnea may not provide sufficiently-early signaling of elevated atrial pressure. By monitoring pressure directly according to examples of the present disclosure, normal ventricular filling pressures may advantageously be maintained, thereby preventing or reducing effects of heart failure, such as dyspnea.

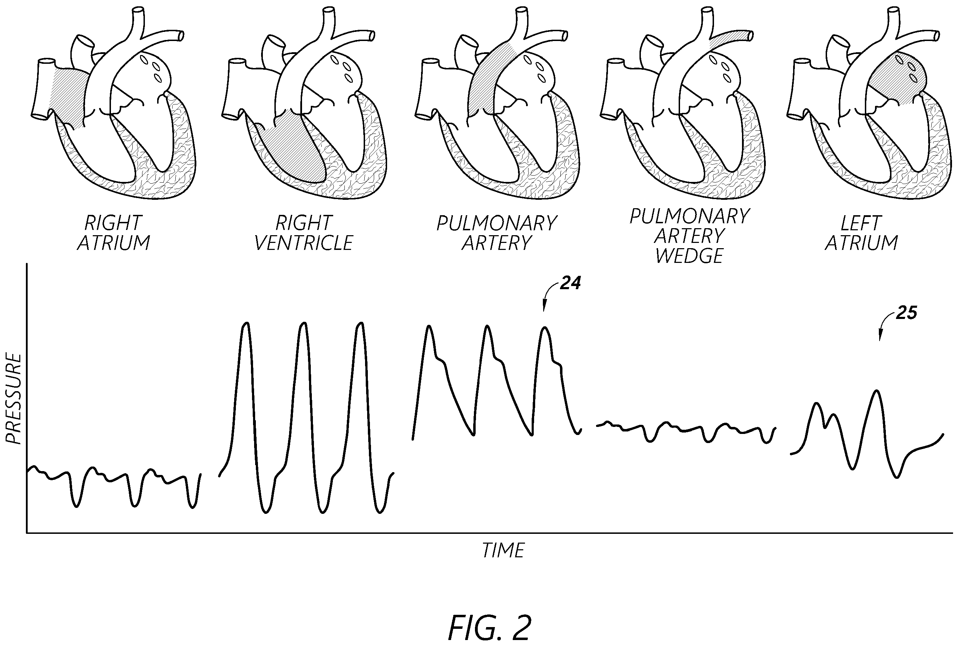

As referenced above, with respect to cardiac pressures, pressure elevation in the left atrium may be particularly correlated with heart failure. Figure 2 illustrates example pressure waveforms associated with various chambers and vessels of the heart according to one or more examples. The various waveforms illustrated in Figure 2 may represent waveforms obtained using right heart catheterization to advance one or more pressure sensors to the respective illustrated and labeled chambers or vessels of the heart. As illustrated in Figure 2, the waveform 25, which represents left atrial pressure, may be considered to provide the best feedback for early detection of congestive heart failure. Furthermore, there may generally be a relatively strong correlation between increases and left atrial pressure and pulmonary congestion.

Left atrial pressure may generally correlate well with left ventricular end-diastolic pressure. However, although left atrial pressure and end-diastolic pulmonary artery pressure can have a significant correlation, such correlation may be weakened when the pulmonary vascular resistance becomes elevated. That is, pulmonary artery pressure generally fails to correlate adequately with left ventricular end-diastolic pressure in the presence of a variety of acute conditions, which may include certain patients with congestive heart failure. For example, pulmonary hypertension, which affects approximately 25% to 83% of patients with heart failure, can affect the reliability of pulmonary artery pressure measurement for estimating left-sided filling pressure. Therefore, pulmonary artery pressure measurement alone, as represented by the waveform 24, may be an insufficient or inaccurate indicator of left ventricular end-diastolic pressure, particularly for patients with comorbidities, such as lung disease and/or thromboembolism. Left atrial pressure may further be correlated at least partially with the presence and/or degree of mitral regurgitation.

Left atrial pressure readings may be relatively less likely to be distorted or affected by other conditions, such as respiratory conditions or the like, compared to the other pressure waveforms shown in Figure 2. Generally, left atrial pressure may be significantly predictive of heart failure, such as up two weeks before manifestation of heart failure. For example, increases in left atrial pressure, and both diastolic and systolic heart failure, may occur weeks prior to hospitalization, and therefore knowledge of such increases may be used to predict the onset of congestive heart failure, such as acute debilitating symptoms of congestive heart failure.

Cardiac pressure monitoring, such as left atrial pressure monitoring, can provide a mechanism to guide administration of medication to treat and/or prevent congestive heart failure. Such treatments may advantageously reduce hospital readmissions and morbidity, as well as provide other benefits. An implanted pressure sensor in accordance with examples of the present disclosure may be used to predict heart failure up two weeks or more before the manifestation of symptoms or markers of heart failure (e.g., dyspnea). When heart failure predictors are recognized using cardiac pressure sensor examples in accordance with the present disclosure, certain prophylactic measures may be implemented, including medication intervention, such as modification to a patient's medication regimen, which may help prevent or reduce the effects of cardiac dysfunction. Direct pressure measurement in the left atrium can advantageously provide an accurate indicator of pressure buildup that may lead to heart failure or other complications. For example, trends of atrial pressure elevation may be analyzed or used to determine or predict the onset of cardiac dysfunction, wherein drug or other therapy may be augmented to cause reduction in pressure and prevent or reduce further complications.

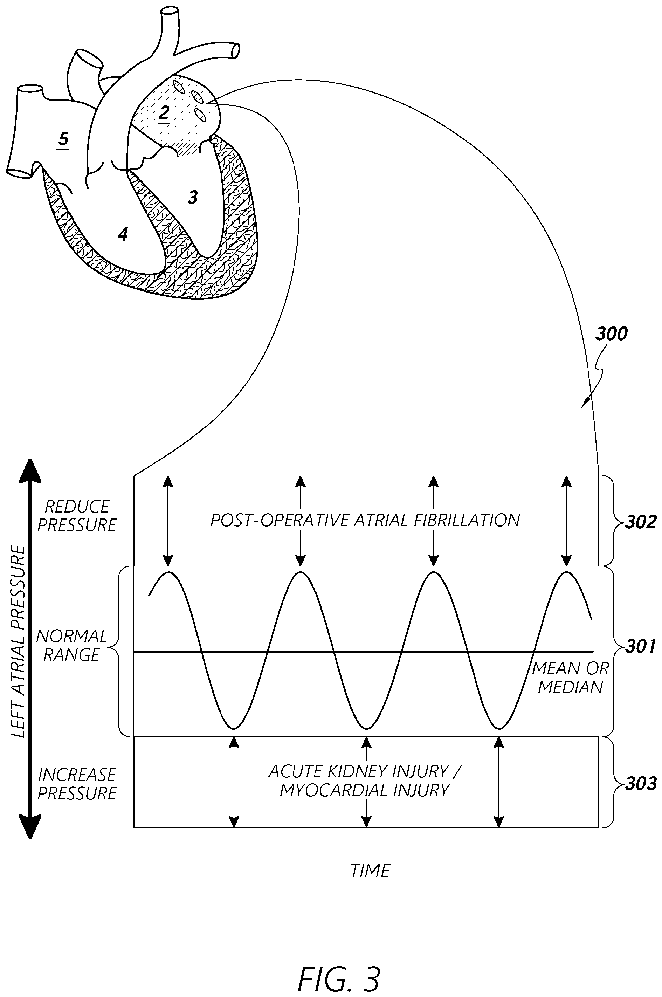

Figure 3 illustrates a graph 300 showing left atrial pressure ranges including a normal range 301 of left atrial pressure that is not generally associated with substantial risk of postoperative atrial fibrillation, acute kidney injury, myocardial injury, heart failure and/or other health conditions. Examples of the present disclosure provide systems, devices, and methods for determining whether a patient's left atrial pressure is within the normal range 301, above the normal range 303, or below the normal range 302 through the use of certain sensor implant devices. For detected left atrial pressure above the normal range, which may be correlated with an increased risk of heart failure, examples of the present disclosure as described in detail below can inform efforts to reduce the left atrial pressure until it is brought within the normal range 301. Furthermore, for detected left atrial pressure that is below the normal range 301, which may be correlated with increased risks of acute kidney injury, myocardial injury, and/or other health complications, examples of the present disclosure as described in detail below can serve to facilitate efforts to increase the left atrial pressure to bring the pressure level within the normal range 301.

### Implant Devices with Integrated Sensors

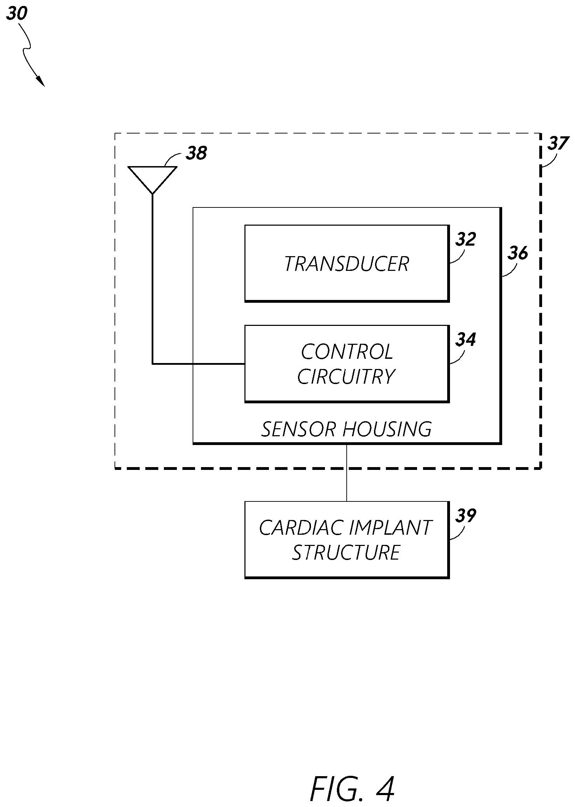

**In** some implementations, the present disclosure relates to sensors associated or integrated with cardiac shunts or other implant devices. Such integrated devices may be used to provide controlled and/or more effective therapies for treating and preventing heart failure and/or other health complications related to cardiac function. Figure 4 is a block diagram illustrating an implant device 30 comprising a shunt (or other type of implant) structure 39. **In** some examples, the shunt structure 39 is physically integrated with and/or connected to a sensor device 37. The sensor device 37 may be, for example, a pressure sensor, or other type of sensor. **In** some examples, the sensor 37 comprises a transducer 32, such as a pressure transducer, as well as certain control circuitry 34, which may be embodied in, for example, an application-specific integrated circuit (ASIC).

The control circuitry 34 may be configured to process signals received from the transducer 32 and/or communicate signals associated therewith wirelessly through biological tissue using the antenna 38. The term "control circuitry" is used herein according to its broad and ordinary meaning, and may refer to any collection of processors, processing circuitry, processing modules/units, chips, dies (e.g., semiconductor dies including come or more active and/or passive devices and/or connectivity circuitry), microprocessors, microcontrollers, digital signal processors, microcomputers, central processing units, field programmable gate arrays, programmable logic devices, state machines (e.g., hardware state machines), logic circuitry, analog circuitry, digital circuitry, and/or any device that manipulates signals (analog and/or digital) based on hard coding of the circuitry and/or operational instructions. Control circuitry referenced herein may further comprise one or more, storage devices, which may be embodied in a single memory device, a plurality of memory devices, and/or embedded circuitry of a device. Such data storage may comprise read-only memory, random access memory, volatile memory, non-volatile memory, static memory, dynamic memory, flash memory, cache memory, data storage registers, and/or any device that stores digital information. It should be noted that in examples in which control circuitry comprises a hardware and/or software state machine, analog circuitry, digital circuitry, and/or logic circuitry, data storage device(s)/register(s) storing any associated operational instructions may be embedded within, or external to, the circuitry comprising the state machine, analog circuitry, digital circuitry, and/or logic circuitry. The transducer(s) 32 and/or antenna(s) 38 can be considered part of the control circuitry 34.

The antenna 38 may comprise one or more coils or loops of conductive material, such as copper wire or the like. **In** some examples, at least a portion of the transducer 32, control circuitry 34, and/or the antenna 38 are at least partially disposed or contained within a sensor housing 36, which may comprise any type of material, and may advantageously be at least partially hermetically sealed. For example, the housing 36 may comprise glass or other rigid material in some examples, which may provide mechanical stability and/or protection for the components housed therein. **In** some examples, the housing 36 is at least partially flexible. For example, the housing may comprise polymer or other flexible structure/material, which may advantageously allow for folding, bending, or collapsing of the sensor 37 to allow for transportation thereof through a catheter or other introducing means.

The transducer 32 may comprise any type of sensor means or mechanism. For example, the transducer 32 may be a force-collector-type pressure sensor. **In** some examples, the transducer 32 comprises a diaphragm, piston, bourdon tube, bellows, or other strain- or deflection-measuring component(s) to measure strain or deflection applied over an area/surface thereof. The transducer 32 may be associated with the housing 36, such that at least a portion thereof is contained within or attached to the housing 36. With respect to sensor devices/components being "associated with" a stent or other implant structure, such terminology may refer to a sensor device or component being physically coupled, attached, or connected to, or integrated with, the implant structure.

**In** some examples, the transducer 32 comprises or is a component of a piezoresistive strain gauge, which may be configured to use a bonded or formed strain gauge to detect strain due to applied pressure, wherein resistance increases as pressure deforms the component/material. The transducer 32 may incorporate any type of material, including but not limited to silicon (e.g., monocrystalline), polysilicon thin film, bonded metal foil, thick film, silicon-on-sapphire, sputtered thin film, and/or the like.

**In** some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicon, and the like. **In** some examples, the transducer 32 comprises or is a component of an electromagnetic pressure sensor, which may be configured to measure the displacement of a diaphragm by means of changes in inductance, linear variable displacement transducer (LVDT) functionality, Hall Effect, or eddy current sensing. **In** some examples, the transducer 32 comprises or is a component of a piezoelectric strain sensor. For example, such a sensor may determine strain (e.g., pressure) on a sensing mechanism based on the piezoelectric effect in certain materials, such as quartz.

**In** some examples, the transducer 32 comprises or is a component of a strain gauge. For example, a strain gauge example may comprise a pressure sensitive element on or associated with an exposed surface of the transducer 32. **In** some examples, a metal strain gauge is adhered to a surface of the sensor, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

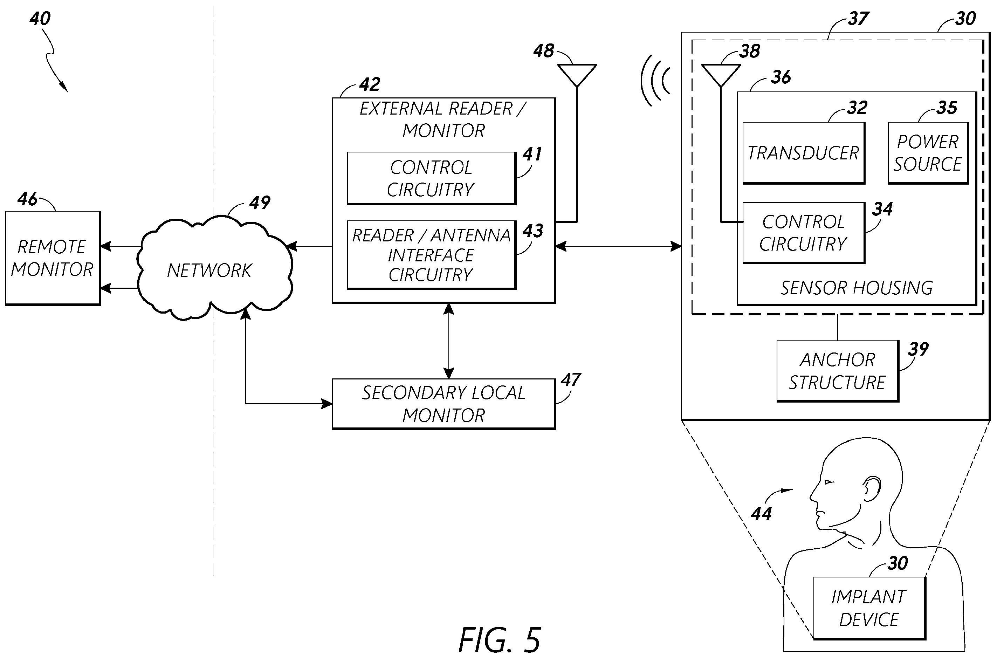

Figure 5 shows a system 40 for monitoring one or more physiological parameters (e.g., left atrial pressure and/or volume) in a patient 44 according to one or more examples. The patient 44 can have a medical implant device 30 implanted in, for example, the heart (not shown), or associated physiology, of the patient 44. For example, the implant device 30 can be implanted at least partially within the left atrium and/or coronary sinus of the patient's heart. The implant device 30 can include one or more sensor transducers 32, such as one or more microelectromechanical system (MEMS) devices (e.g., MEMS pressure sensors, or other type of sensor transducer).

In certain examples, the monitoring system 40 can comprise at least two subsystems, including an implantable internal subsystem or device 30 that includes the sensor transducer(s) 32, as well as control circuitry 34 comprising one or more microcontroller(s), discrete electronic component(s), and one or more power and/or data transmitter(s) 38 (e.g., antennae coil). The monitoring system 40 can further include an external (e.g., non-implantable) subsystem that includes an external reader 42 (e.g., coil), which may include a wireless transceiver that is electrically and/or communicatively coupled to certain control circuitry 41. In certain examples, both the internal 30 and external 42 subsystems include a corresponding coil antenna for wireless communication and/or power delivery through patient tissue disposed therebetween. The sensor implant device 30 can be any type of implant device. For example, in some examples, the implant device 30 comprises a pressure sensor integrated with another functional implant structure 39, such as a prosthetic shunt or stent device/structure.

Certain details of the implant device 30 are illustrated in the enlarged block 30 shown. The implant device 30 can comprise an implant/anchor structure 39 as described herein. For example, the implant/anchor structure 39 can include a percutaneously-deliverable shunt device configured to be secured to and/or in a tissue wall to provide a flow path between two chambers and/or vessels of the heart, as described in detail throughout the present disclosure. Although certain components are illustrated in Figure 5 as part of the implant device 30, it should be understood that the sensor implant device 30 may only comprise a subset of the illustrated components/modules and can comprise additional components/modules not illustrated. The implant device may represent an example of the implant device shown in Figure 4, and vice versa. The implant device 30 can advantageously include one or more sensor transducers 32, which can be configured to provide a response indicative of one or more physiological parameters of the patient 44, such as atrial pressure. Although pressure transducers are described, the sensor transducer(s) 32 can comprise any suitable or desirable types of sensor transducer(s) for providing signals relating to physiological parameters or conditions associated with the implant device 30 and/or patient 44.

The sensor transducer(s) 32 can comprise one or more MEMS sensors, optical sensors, piezoelectric sensors, electromagnetic sensors, strain sensors/gauges, accelerometers, gyroscopes, diaphragm-based sensors, and/or other types of sensors, which can be positioned in the patient 44 to sense one or more parameters relevant to the health of the patient. The transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the sensor housing 36, such that at least a portion thereof is contained within, or attached to, the housing 36.

In some examples, the transducer 32 comprises or is a component of a strain gauge, which may be configured to use a bonded or formed strain gauge to detect strain due to applied pressure. For example, the transducer 32 may comprise or be a component of a piezoresistive strain gauge, wherein resistance increases as pressure deforms the component/material of the strain gauge. The transducer 32 may incorporate any type of material, including but not limited to silicone, polymer, silicon (e.g., monocrystalline), polysilicon thin film, bonded metal foil, thick film, silicon-on-sapphire, sputtered thin film, and/or the like. In some examples, a metal strain gauge is adhered to the sensor surface, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

**In** some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicone, silicon or other semiconductor, and the like. **In** some examples, the transducer 32 comprises or is a component of an electromagnetic pressure sensor, which may be configured to measures the displacement of a diaphragm by means of changes in inductance, linear variable displacement transducer (LVDT) functionality, Hall Effect, or eddy current sensing. **In** some examples, the transducer 32 comprises or is a component of a piezoelectric strain sensor. For example, such a sensor may determine strain (e.g., pressure) on a sensing mechanism based on the piezoelectric effect in certain materials, such as quartz.

In some examples, the transducer(s) 32 is/are electrically and/or communicatively coupled to the control circuitry 34, which may comprise one or more application-specific integrated circuit (ASIC) microcontrollers or chips. The control circuitry 34 can further include one or more discrete electronic components, such as tuning capacitors, resistors, diodes, inductors, or the like.

In certain examples, the sensor transducer(s) 32 can be configured to generate electrical signals that can be wirelessly transmitted to a device outside the patient's body, such as the illustrated local external monitor system 42. In order to perform such wireless data transmission, the implant device 30 can include radio frequency (RF) (or other frequency band) transmission circuitry, such as signal processing circuitry and an antenna 38. The antenna 38 can comprise an antenna coil implanted within the patient. The control circuitry 34 may comprise any type of transceiver circuitry configured to transmit an electromagnetic signal, wherein the signal can be radiated by the antenna 38, which may comprise one or more conductive wires, coils, plates, or the like. The control circuitry 34 of the implant device 30 can comprise, for example, one or more chips or dies configured to perform some amount of processing on signals generated and/or transmitted using the device 30. However, due to size, cost, and/or other constraints, the implant device 30 may not include independent processing capability in some examples.

The wireless signals generated by the implant device 30 can be received by the local external monitor device or subsystem 42, which can include a reader/antenna-interface circuitry module 43 configured to receive the wireless signal transmissions from the implant device 30, which is disposed at least partially within the patient 44. For example, the module 43 may include transceiver device(s)/circuitry.

The external local monitor 42 can receive the wireless signal transmissions from the implant device 30 and/or provide wireless power to the implant device 30 using an external antenna 48, such as a wand device. The reader/antenna-interface circuitry 43 can include radio-frequency (RF) (or other frequency band) front-end circuitry configured to receive and amplify the signals from the implant device 30, wherein such circuitry can include one or more filters (e.g., band-pass filters), amplifiers (e.g., low-noise amplifiers), analog-to-digital converters (ADC) and/or digital control interface circuitry, phase-locked loop (PLL) circuitry, signal mixers, or the like. The reader/antenna-interface circuitry 43 can further be configured to transmit signals over a network 49 to a remote monitor subsystem or device 46. The RF circuitry of the reader/antenna-interface circuitry 43 can further include one or more of digital-to-analog converter (DAC) circuitry, power amplifiers, low-pass filters, antenna switch modules, antennas or the like for treatment/processing of transmitted signals over the network 49 and/or for receiving signals from the implant device 30. In certain examples, the local monitor 42 includes control circuitry 41 for performing processing of the signals received from the implant device 30. The local monitor 42 can be configured to communicate with the network 49 according to a known network protocol, such as Ethernet, Wi-Fi, or the like. In certain examples, the local monitor 42 comprises a smartphone, laptop computer, or other mobile computing device, or any other type of computing device.

In certain examples, the implant device 30 includes some amount of volatile and/or non-volatile data storage. For example, such data storage can comprise solid-state memory utilizing an array of floating-gate transistors, or the like. The control circuitry 34 may utilize data storage for storing sensed data collected over a period of time, wherein the stored data can be transmitted periodically to the local monitor 42 or other external subsystem. In certain examples, the implant device 30 does not include any data storage. The control circuitry 34 may be configured to facilitate wireless transmission of data generated by the sensor transducer(s) 32, or other data associated therewith. The control circuitry 34 may further be configured to receive input from one or more external subsystems, such as from the local monitor 42, or from a remote monitor 46 over, for example, the network 49. For example, the implant device 30 may be configured to receive signals that at least partially control the operation of the implant device 30, such as by activating/deactivating one or more components or sensors, or otherwise affecting operation or performance of the implant device 30.

The one or more components of the implant device 30 can be powered by one or more power sources 35. Due to size, cost and/or electrical complexity concerns, it may be desirable for the power source 35 to be relatively minimalistic in nature. For example, high-power driving voltages and/or currents in the implant device 30 may adversely affect or interfere with operation of the heart or other body part associated with the implant device. In certain examples, the power source 35 is at least partially passive in nature, such that power can be received from an external source wirelessly by passive circuitry of the implant device 30, such as through the use of short-range, or near-field wireless power transmission, or other electromagnetic coupling mechanism. For example, the local monitor 42 may serve as an initiator that actively generates an RF field that can provide power to the implant device 30, thereby allowing the power circuitry of the implant device to take a relatively simple form factor. In certain examples, the power source 35 can be configured to harvest energy from environmental sources, such as fluid flow, motion, or the like. Additionally or alternatively, the power source 35 can comprise a battery, which can advantageously be configured to provide enough power as needed over the monitoring period (e.g., 3, 5, 10, 20, 30, 40, or 90 days, or any other period of time).

In some examples, the local monitor device 42 can serve as an intermediate communication device between the implant device 30 and the remote monitor 46. The local monitor device 42 can be a dedicated external unit designed to communicate with the implant device 30. For example, the local monitor device 42 can be a wearable communication device, or other device that can be readily disposed in proximity to the patient 44 and implant device 30. The local monitor device 42 can be configured to continuously, periodically, or sporadically interrogate the implant device 30 in order to extract or request sensor-based information therefrom. In certain examples, the local monitor 42 comprises a user interface, wherein a user can utilize the interface to view sensor data, request sensor data, or otherwise interact with the local monitor system 42 and/or implant device 30.

The system 40 can include a secondary local monitor 47, which can be, for example, a desktop computer or other computing device configured to provide a monitoring station or interface for viewing and/or interacting with the monitored cardiac pressure data. In an example, the local monitor 42 can be a wearable device or other device or system configured to be disposed in close physical proximity to the patient and/or implant device 30, wherein the local monitor 42 is primarily designed to receive/transmit signals to and/or from the implant device 30 and provide such signals to the secondary local monitor 47 for viewing, processing, and/or manipulation thereof. The external local monitor system 42 can be configured to receive and/or process certain metadata from or associated with the implant device 30, such as device ID or the like, which can also be provided over the data coupling from the implant device 30.

The remote monitor subsystem 46 can be any type of computing device or collection of computing devices configured to receive, process and/or present monitor data received over the network 49 from the local monitor device 42, secondary local monitor 47, and/or implant device 30. For example, the remote monitor subsystem 46 can advantageously be operated and/or controlled by a healthcare entity, such as a hospital, doctor, or other care entity associated with the patient 44. Although certain examples disclosed herein describe communication with the remote monitor subsystem 46 from the implant device indirectly through the local monitor device 42, in certain examples, the implant device 30 can comprise a transmitter capable of communicating over the network 49 with the remote monitor subsystem 46 without the necessity of relaying information through the local monitor device 42.

In some examples, at least a portion of the transducer 32, control circuitry 34, power source 35 and/or the antenna 38 are at least partially disposed or contained within the sensor housing 36, which may comprise any type of material, and may advantageously be at least partially hermetically sealed. For example, the housing 36 may comprise glass or other rigid material in some examples, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other percutaneous introducing means.

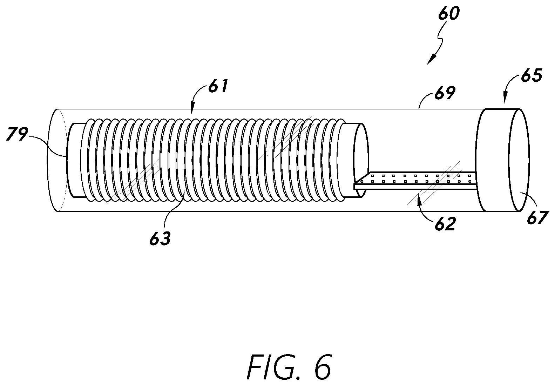

As referenced above, shunt and other implant devices/structures may be integrated with sensor, antenna/transceiver, and/or other components to facilitate in vivo monitoring of pressure and/or other physiological parameter(s). Sensor devices in accordance with examples of the present disclosure may be integrated with cardiac shunt structures/devices or other implant devices using any suitable or desirable attachment or integration mechanism or configuration. Figure 6 illustrates an example sensor assembly/device 60 that can be a component of a sensor implant device. The sensor device 60 may be configured to provide sensor readings relating to one or more physiological parameters associated with a target implantation site.

The sensor device 60 may be configured for attachment to implant devices. For example, a coil form including one or more wires or other material or structure shaped into one or more winds of coil forming a fluid conduit/barrel portion and axial end flanges may be used to attach the sensor device 60 to one or more implants. A shunt structure may be integrated with pressure sensor functionality in accordance with certain examples disclosed herein. The shunt structure may be configured to hold the sensor device 60.

The sensor device 60 may advantageously be disposed, positioned, secured, oriented, and/or otherwise situated in a configuration in which a sensor transducer component 65 thereof is disposed within a channel area of a shunt structure. The term "channel area" is used herein according to its broad and ordinary meaning and may refer to a three-dimensional space defined by a radial boundary of a fluid conduit and extending axially from the fluid conduit.

In some examples, the sensor assembly 61 includes a sensor component 65 and an antenna component 69. The sensor component 65 may comprise any type of sensor device as described in detail above. In some examples, the sensor 65 may be attached to or integrated with an arm member of a shunt structure.

The sensor 65 includes a sensor element 67, such as a pressure sensor transducer. As described herein, the sensor assembly 61 may be configured to implement wireless data and/or power transmission. The sensor assembly 61 may include an antenna component 69 for such purpose. The antenna 69 may be contained at least partially within an antenna housing 79, which may further have disposed therein certain control circuitry configured to facilitate wireless data and/or power communication functionality. In some examples, the antenna component 69 comprises one or more conductive coils 62, which may facilitate inductive powering and/or data transmission. In examples comprising conductive coil(s), such coil(s) may be wrapped/disposed at least partially around a magnetic (e.g., ferrite, iron) core 63.

The antenna component 69 may be attached to, integrated with, or otherwise associated with an arm/anchor feature of a shunt structure

The sensor assembly 61 may advantageously be biocompatible. For example, the sensor 65 and antenna 69 may comprise biocompatible housings, such as a housing comprising glass or other biocompatible material. However, at least a portion of the sensor element 67, such as a diaphragm or other component, may be exposed to the external environment in some examples in order to allow for pressure readings, or other parameter sensing, to be implemented. With respect to the antenna housing 79, the housing 79 may comprise an at least partially rigid cylindrical or tube-like form, such as a glass cylinder form. In some examples, the sensor 65/67 component is approximately 3 mm or less in diameter. The antenna 69 may be approximately 20 mm or less in length.

The sensor assembly 61 may be configured to communicate with an external system when implanted in a heart or other area of a patient's body. For example, the antenna 69 may receive power wirelessly from the external system and/or communicate sensed data or waveforms to and/or from the external system. The sensor assembly 61 may be attached to, or integrated with, a shunt structure in any suitable or desirable way. For example, in some implementations, the sensor 65 and/or antenna 69 may be attached or integrated with the shunt structure using mechanical attachment means. In some examples, the sensor 65 and/or antenna 69 may be contained in a pouch or other receptacle that is attached to a shunt structure.

The sensor element 67 may comprise a pressure transducer. For example, the pressure transducer may be a microelectromechanical system (MEMS) transducer comprising a semiconductor diaphragm component. In some examples, the transducer may include an at least partially flexible or compressible diaphragm component, which may be made from silicone or other flexible material. The diaphragm component may be configured to be flexed or compressed in response to changes in environmental pressure.

### Cardiac Implants

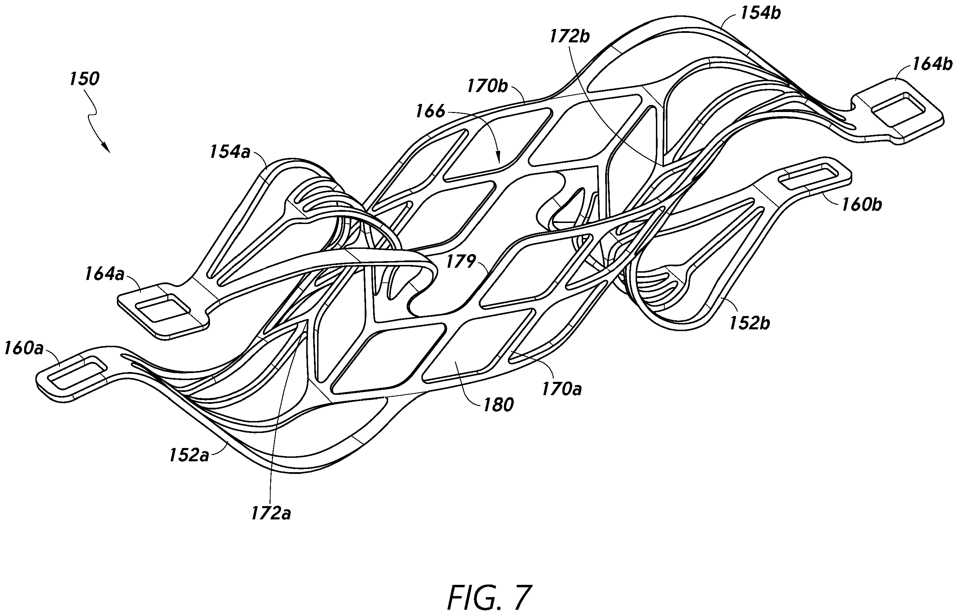

Figure 7 illustrates an example shunt/anchor structure 150 which may be configured for attachment to one or more sensor devices, in accordance with one or more examples. The shunt structure 150 may represent an example of a cardiac implant (e.g., anchor and/or cardiac implant structure associated with Figure 4 or 5) that may be integrated with pressure sensor functionality in accordance with certain examples disclosed herein. The shunt structure 150 may be an expandable shunt. When expanded, a central flow channel 166 of the shunt 150 may define a generally circular or oval-shaped opening and/or may form a fluid conduit when positioned within an orifice of a tissue wall. The channel 166 may be configured to hold the sides of a puncture opening and/or other orifice in a tissue wall to form a blood flow path between chamber(s) or vessel(s) of the heart that are separated by the tissue wall. For example, the shunt 150 may be configured to be implanted in the wall separating the coronary sinus and the left atrium to form a fluid conduit between the coronary sinus and the left atrium. The central flow channel 166 may be partly formed by a pair of side walls 170a, 170b defined by a generally parallel arrangement of thin struts 179 that forms an array of parallelogram-shaped cells or openings 180. In some examples, substantially the entire shunt 150 is formed by super-elastic struts that are configured to be compressed and fit into a catheter (not shown) and subsequently expanded back to the relaxed shape as shown in Figure 7.

Formation of the shunt 150 using a plurality of interconnected struts forming cells therebetween may serve to at least partially increase the flexibility of the shunt, thereby enabling compression thereof and expansion at the implant site. The interconnected struts around the central flow channel 166 advantageously provide a cage having sufficient rigidity and structure to hold the tissue at the puncture in an open position. End walls 172a, 172b of the central flow channel 166 can serve to connect the side walls 170a, 170b and extend between distal and proximal flanges, or arms, 152, 154 on each side. The side walls 170a, 170b and end walls 172a, 172b together may define a tubular lattice, as shown. The end walls 172a, 172b can comprise thin struts 179 extending at a slight angle from a central flow axis of the shunt 150. The shunt 150 can further comprise terminal ends (160a, 164a, 160b, 164b) of the arms 152, 154 which can be closer together than the ends connected to end walls 172a, 172b of the arms 152, 154.

Although the illustrated shunt 150 comprises struts that define a tubular or circular lattice of open cells forming the central flow channel 166, in some examples, the structure that makes up the channel forms a substantially contiguous wall surface through at least a portion of the channel 166. In the illustrated example, the tilt of the shunt structure 150 may facilitate collapse of the shunt into a delivery catheter (not shown), as well as the expansion of the flanges/arm 152, 154 on both sides of a target tissue wall. The shunt 150 may comprise a first left arm 152a, a second left arm 154a, a first right arm 152b, and/or a second right arm 154b. The central flow channel 166 may remain essentially unchanged between the collapsed and expanded states of the shunt 150, whereas the flanges/arms 152, 154 may transition in and out of alignment with the angled flow channel.

Although certain examples of shunts disclosed herein comprise flow channels and/or fluid conduits having substantially circular cross-sections, in some examples, shunt structures in accordance with the present disclosure have oval-shaped, rectangular, diamond-shaped, or elliptical flow channel configuration. For example, relatively elongated side walls compared to the illustrated configuration of Figure 7 may produce a rectangular or oval-shaped flow channel. Such shapes of shunt flow channels may be desirable for larger punctures, while still being configured to collapse down to a relatively small delivery profile.

In some examples, each of the distal and proximal flanges/arms 152, 154 is configured to curl outward from the end walls 172a, 172b and be set to point approximately radially away from the central flow channel 166 in the expanded configuration. The expanded flanges/arms may serve to secure the shunt 150 to a target tissue wall. Additional aspects and features of shunt, implant, and/or anchor structures that may be integrated with sensor devices/functionality of examples of the present disclosure are disclosed in U.S. Pat. No. 9,789,294, entitled "Expandable Cardiac Shunt," issued on October 17, 2017, the disclosure of which is hereby expressly incorporated by reference in its entirety. Although certain examples are disclosed herein in the context of shunt structures similar to that shown in Figure 7 and described above, it should be understood that shunt structures or other implant devices integrated with pressure sensor functionality in accordance with examples of the present disclosure may have any type, form, structure, configuration, and/or may be used or configured to be used for any purpose, whether for shunting or other purpose or functionality.

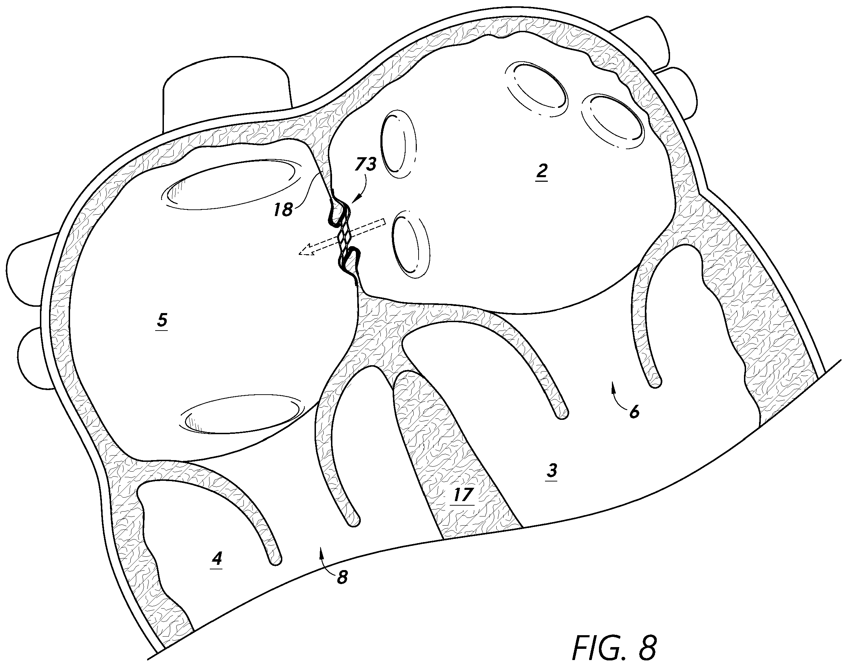

Figure 8 shows a shunt implant/anchor device/structure 73 implanted in an atrial septum 18 in accordance with one or more examples. While a shunt implant is depicted in Figure 8, the implant 73 may be any of the various implants described herein. The particular position in the interatrial septum wall 18 may be selected or determined to provide a relatively secure anchor location for the shunt structure 73. Furthermore, the shunt device/structure 73 may be implanted at a position that is desirable in consideration of future re-crossing of the septal wall 18 for future interventions. Implantation of the shunt device/structure 73 in the interatrial septum wall 18 may advantageously allow for fluid communication between the left 2 and right 5 atria.

Interatrial shunting using the shunt device/structure 73 may be well-suited for patients that are relatively highly sensitive to atrial pressure increases. For example, as pressure increases in the ventricles and/or atria and is applied against the myocardial cells, the muscles of the heart may generally be prone to contract relatively harder to process the excess blood. Therefore, as the ventricle dilates or stretches, for patients with compromised contractility of the ventricle, such patients may become more sensitive to higher pressures in the ventricle and/or atria because the heart may be unable to adequately respond or react thereto. Furthermore, increases in left atrial pressure can results in dyspnea, and therefore reduction in left atrial pressure to reduce dyspnea and/or reduce incidences of hospital readmission may be desirable through interatrial shunting. For example, when the ventricle experiences dysfunction such that is unable to accommodate build-up in fluid pressure, such fluid may backup into the atria, thereby increasing atrial pressure. With respect to heart failure, minimization of left ventricular end-diastolic pressure may be paramount. Because left ventricular end-diastolic pressure can be related to left atrial pressure, backup of fluid in the atrium can cause backup of fluid in the lungs, thereby causing undesirable and/or dangerous fluid buildup in the lungs. Interatrial shunting, such as using shunt devices in accordance with examples of the present disclosure, can divert extra fluid in the left atrium to the right atrium, which may be able to accommodate the additional fluid due to the relatively high compliance in the right atrium.

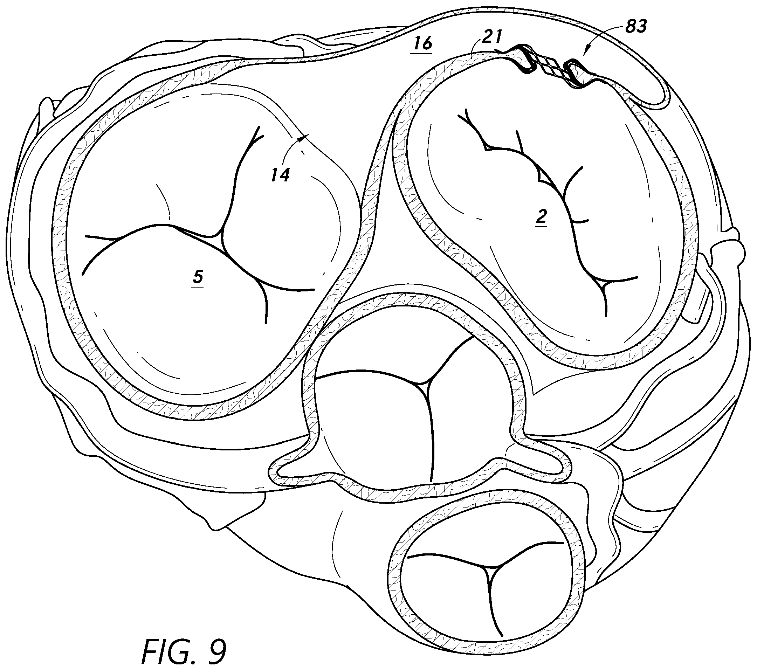

In some implementations, shunt device/structure in accordance with examples of the present disclosure may be implanted in a wall separating the coronary sinus from the left atrium, such that interatrial shunting may be achieved through the coronary sinus. Figure 9 shows a shunt device/structure 83 implanted in a tissue wall 21 between the coronary sinus 16 and the left atrium 2. While a shunt implant is depicted in Figure 9, the implant 83 may be any of the various implants described herein. Figure 9, as well as a number of the following figures, shows a section of the heart from a top-down, superior perspective with the posterior aspect oriented at the top of the page.

In some cases, left-to-right shunting through implantation of the shunt device 83 in the wall 21 between the left atrium 2 and the coronary sinus 16 can be preferable to shunting through the interatrial septum. For example, shunting through the coronary sinus 16 can provide reduced risk of thrombus and embolism. The coronary sinus is less likely to have thrombus/emboli present for several reasons. First, the blood draining from the coronary vasculature into the right atrium 5 has just passed through capillaries, so it is essentially filtered blood. Second, the ostium 14 of the coronary sinus in the right atrium is often partially covered by a pseudo-valve called the Thebesian Valve (not shown). The Thebesian Valve is not always present, but some studies show it is present in most hearts and can block thrombus or other emboli from entering in the event of a spike in right atrium pressure. Third, the pressure gradient between the coronary sinus and the right atrium into which it drains is generally relatively low, such that thrombus or other emboli in the right atrium is likely to remain there. Fourth, in the event that thrombus/emboli do enter the coronary sinus, there will be a much greater gradient between the right atrium and the coronary vasculature than between the right atrium and the left atrium. Most likely, thrombus/emboli would travel further down the coronary vasculature until right atrium pressure returned to normal and then the emboli would return directly to the right atrium.

Some additional advantages to locating the shunt structure 83 between the left atrium and the coronary sinus is that this anatomy is generally more stable than the interatrial septal tissue. By diverting left atrial blood into the coronary sinus, sinus pressures may increase by a small amount. This would cause blood in the coronary vasculature to travel more slowly through the heart, increasing perfusion and oxygen transfer, which can be more efficient and also can help a dying heart muscle to recover. **In** addition, by implanting the shunt device/structure 83 in the wall of the coronary sinus, damage to the interatrial septum 18 may be prevented. Therefore, the interatrial septum 18 may be preserved for later transseptal access for alternate therapies. The preservation of transseptal access may be advantageous for various reasons. For example, heart failure patients often have a number of other comorbidities, such as atrial fibrillation and/or mitral regurgitation; certain therapies for treating these conditions require a transseptal access.

It should be noted, that in addition to the various benefits of placing the implant/structure 83 between the coronary sinus 16 and the left atrium 2, certain drawbacks may be considered. For example, by shunting blood from the left atrium 2 to the coronary sinus 16, oxygenated blood from the left atrium 2 may be passed to the right atrium 5 and/or non-oxygenated blood from the right atrium 5 may be passed to the left atrium 2, both of which may be undesirable with respect to proper functioning of the heart.

### Sensor Implant Devices

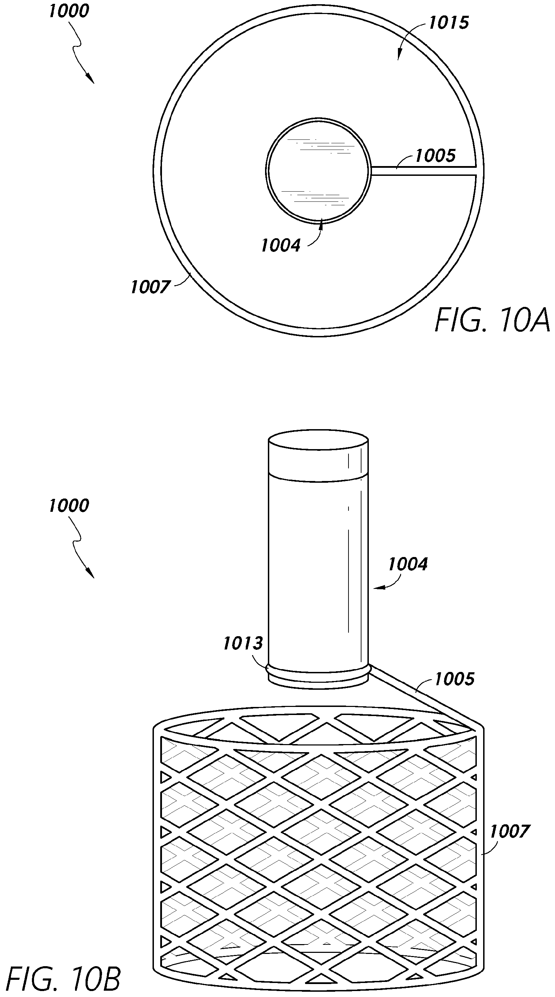

Figures 10A and 10B illustrate example sensor implant devices 1000 in accordance with one or more examples of the present disclosure. Figure 10A provides an overhead view of an example sensor implant device 1000 comprising a sensor 1004 coupled to at least a portion (e.g., a shunt body 1007 and/or barrel portion) of the implant 1000. The term "shunt body" is used herein in accordance with its plain and ordinary meaning and may refer to a body portion of sensor implant device 1000 and/or a portion of a sensor implant device 1000 configured for placement at least partially within a shunt opening (i.e., opening) and/or orifice through a tissue wall. In some examples, the various shunt bodies 1007 described herein may be configured to maintain a shunt opening (e.g., by preventing ingrowth at the opening). A shunt body 1007 can have a generally tubular and/or cylindrical form and/or a partial tube and/or partial cylinder form. Figure 10B provides a side view of the example sensor implant device 1000. Sensors 1004 described herein may be coupled to shunt bodies 1007 in any of a variety of ways. For example, an arm 1005, which can include one or more cords, wires, tethers, and/or similar devices, can extend from the shunt body 1007 and/or sensor device 1004 and/or may be coupled between the shunt body 1007 and the sensor device 1004. In some examples, the arm 1005 may be coupled to and/or may extend into one or more coils 1013 configured to wrap at least partially around at least a portion of the sensor device 1004 to form a secure attachment between the arm 1005 and the sensor device 1004. For example, the arm 1005 may comprise a cord configured to extend between the sensor 1004 and the shunt body 1007 and/or configured to wrap at least partially around the sensor 1004 to form a secure attachment to the sensor 1004. In some examples, the sensor 1004 may be coupled to the shunt body 1007 via one or more arms 1005.

In some examples, sensor implant devices 1000 and/or shunt bodies 1007 described herein may be configured to provide a fluid conduit through an opening and/or orifice at a tissue wall within a heart. For example, the shunt body 1007 may be configured for placement at least partially within an opening in a tissue wall between a coronary sinus and a left atrium of the heart. The opening may be formed through a tissue puncture procedure. In some examples, the shunt body 1007 may be configured to maintain the opening and/or to prevent growth of tissue across the opening. Blood flow between the left atrium and the coronary sinus may be enabled to flow through at least a portion of the shunt body 1007 and/or through the opening.

The sensor device 1004 may be configured to collect any of a variety of measurements related to blood flow at or near the sensor implant device 1000. As shown in Figures 10A and 10B, the sensor device 1004 may be configured to be positioned at least partially over an inner lumen 1015 of the shunt body 1007. For example, the shunt body 1007 may have an at least partial cylindrical form. The sensor device 1004 may be positioned at least partially over the shunt body 1007 such that blood flow through the shunt body 1007 may pass along the sensor device 1004 and/or the sensor device 1004 may be positioned to collect measurements related to blood flow through the shunt body 1007 and/or inner lumen 1015 of the sensor implant device 1000. In some examples, the arm 1005 of the sensor implant device 1000 may be at least partially adjustable to allow for adjustment to the placement of the sensor device 1004 while the sensor device 1004 is coupled at least indirectly to the shunt body 1007.

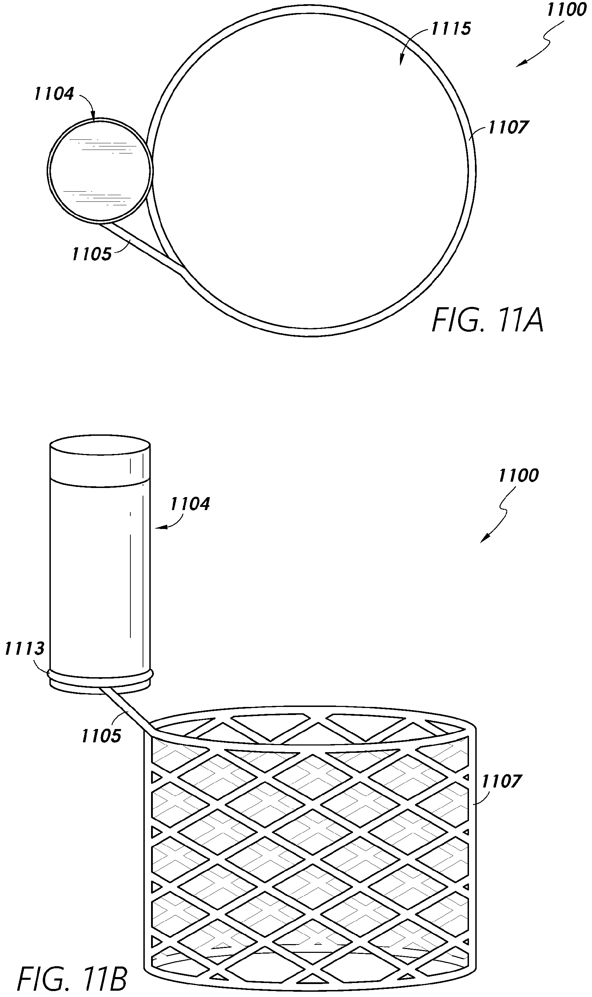

Figures 11A and 11B illustrate example sensor implant devices 1100 in accordance with one or more examples of the present disclosure. Figure 11A provides an overhead view of an example sensor implant device 1100 comprising a sensor 1104 coupled to at least a portion (e.g., a shunt body 1107) of the implant 1100. Figure 11B provides a side view of the example sensor implant device 1100. Sensors 1104 described herein may be coupled to shunt bodies 1107 in any of a variety of ways. For example, an arm 1105, which can include one or more cords, wires, tethers, and/or similar devices, can extend from the shunt body 1107 and/or sensor device 1104 and/or may be coupled between the shunt body 1107 and the sensor device 1104. In some examples, the arm 1105 may be coupled to and/or may extend into one or more coils 1113 configured to wrap at least partially around at least a portion of the sensor device 1104 to form a secure attachment between the arm 1105 and the sensor device 1104. For example, the arm 1105 may comprise a cord configured to extend between the sensor 1104 and the shunt body 1107 and/or configured to wrap at least partially around the sensor 1104 to form a secure attachment to the sensor 1104.

In some examples, sensor implant devices 1100 and/or shunt bodies 1107 described herein may be configured to provide a fluid conduit through an opening and/or orifice at a tissue wall within a heart. For example, the shunt body 1107 may be configured for placement at least partially within an opening in a tissue wall between a coronary sinus and a left atrium of the heart. The opening may be formed through a tissue puncture procedure. In some examples, the shunt body 1107 may be configured to maintain the opening and/or to prevent growth of tissue across the opening. Blood flow between the left atrium and the coronary sinus may be enabled to flow through at least a portion of the shunt body 1107.

The sensor device 1104 may be configured to collect any of a variety of measurements related to blood flow at or near sensor implant device 1100. As shown in Figures 11A and 11B, the sensor device 1104 may be configured to be positioned at least partially offset from an inner lumen 1115 and/or shunt body 1007 of the sensor implant device 1100. For example, the sensor device 1004 may be at least partially outside of a flow pathway of blood through the inner lumen 1115 and/or shunt body 1007 of the sensor implant device 1100 and/or may not be positioned entirely over the inner lumen 1115. Measurements collected by the sensor device 1004 me accordingly be reflective of blood flow characteristics within one of the chambers of the heart and/or may not be directly indicative of blood flow through the sensor implant device 1100. In some examples, the arm 1105 of the sensor implant device 1100 may be at least partially adjustable to allow for adjustment to the placement of the sensor device 1104 while the sensor device 1104 is coupled at least indirectly to the shunt body 1007 (e.g., via one or more arms 1105).

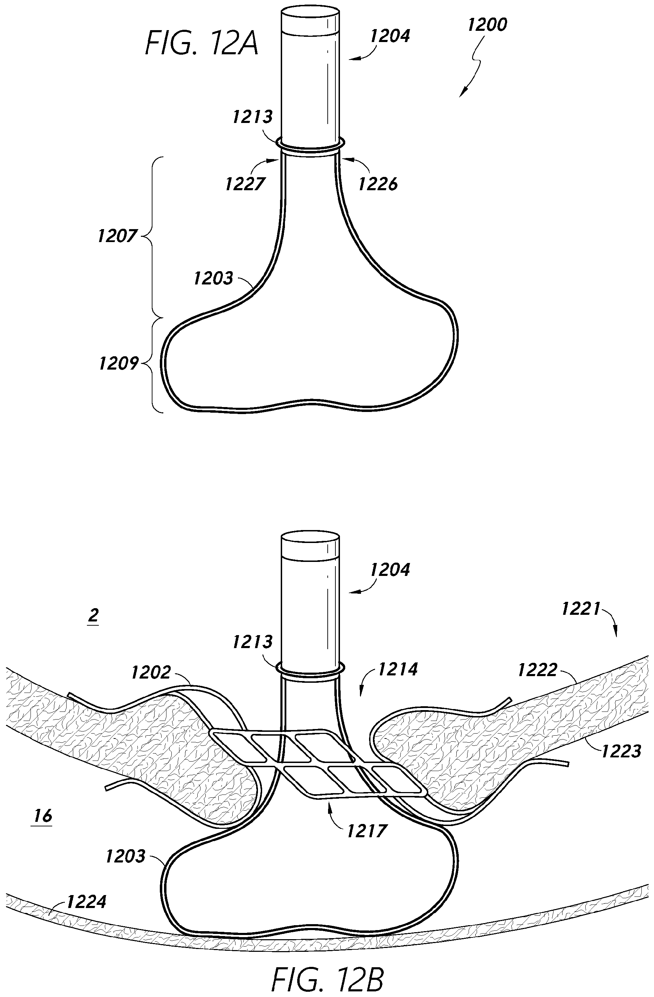

Figures 12A and 12B illustrate a sensor implant device 1200 comprising a sensor 1204 coupled to and/or extending from a shunt body 1203, in accordance with one or more embodiments of the presently claimed invention. The shunt body 1203 may be configured to position the sensor 1204 at a desired position and/or to securely hold the sensor 1204 in place. In some examples, the shunt body 1203 may comprise a wire loop having a first end 1226 configured to couple and/or attach to the sensor 1204 at a first point of the sensor 1204 and/or the shunt body 1203 may comprise a second end 1227 configured to attach and/or couple to the sensor 1204 at a second point of the sensor 1204. The shunt body 1203 has a "mushroom" shape in which a first portion 1207 of the shunt body 1203 has a first width and/or diameter and a second portion 1209 of the shunt body 1203 expands to a second width and/or diameter that is greater than the first width and/or diameter. While the shunt body 1203 is shown in Figures 12A and 12B comprising a single line, the shunt body 1203 may comprise any number of lines and/or may comprise a network of lines forming the mushroom shape depicted in Figures 12A and 12B. For example, the shunt body 1203 may comprise multiple lines which may overlap and/or intersect at various points and/or may expand in different planes with respect to the sensor 1204. In some examples, the shunt body 1203 may form coils 1213 around at least a portion of the sensor 1204.

In some examples, the shunt body 1203 may be at least partially composed of one or more shape-memory alloys (e.g., Nitinol) and/or may be configured to naturally assume the form shown in Figure 12A upon removal from a catheter and/or other delivery device. For example, the shunt body 1203 may comprise one or more wires shape-set into a loop. The wire loop may be configured to assume a compressed form while delivered through a catheter and/or other delivery systems. Moreover, the wire loop is configured to assume the first width at the first portion 1207 ( within a central flow portion 1217 of a shunt device 1202) and is configured to expand to the second width at the second portion 1209 (beyond the central flow portion 1217).

Figure 12B illustrates the sensor implant device 1200 positioned at and/or at least partially within an opening 1214 of a tissue wall 1221. The first portion 1207 of the shunt body 1203 may be configured to be situated at part at least partially within the opening 1214 through the tissue wall 1221. For example, the shunt body 1203 at the first portion 1207 may have a width that is less than a width of the opening 1214 to allow the shunt body 1203 to pass through the opening 1214. The shunt body 1203 at the second portion 1209 may expand laterally (e.g., extend in parallel and/or near-parallel with the tissue wall 1221) to have a width that exceeds the width of the opening 1214. Accordingly, the second portion 1209 of the shunt body 1203 may be configured to prevent the shunt body 1203 from escaping upward through the opening 1214 in the tissue wall 1221. Moreover, the shunt body 1203 at the second portion 1209 may be configured to extend longitudinally (e.g., extend perpendicularly to the tissue wall 1221) and/or away from the sensor 1204 to securely anchor the shunt body 1203 and/or the sensor 1204 in place with respect to the opening of the tissue wall 1221. For example, the second portion 1209 of the one or more anchoring arms may have a suitable height to press against an upper wall 1223 and/or a lower wall 1224 of a coronary sinus 16 and/or other area of the heart to secure the second portion 1209 and/or the sensor implant device 1200 in place in the coronary sinus 16 and/or other area.

While the sensor implant device 1200 may be utilized in some cases independently of other implant devices, the sensor implant device 1200 may additionally or alternatively be used in combination with one or more additional implant devices. In the presently claimed invention, as shown for example in Figure 12B, the sensor implant device 1200 is utilized in combination with a shunt implant 1202 which may be configured to form and/or maintain a fluid conduit through the opening 1214 in the tissue wall 1221. The shunt implant 1202 may comprise one or more anchoring arms configured to anchor to a first side 1222 and/or to a second side (i.e., the upper wall 1223 of the coronary sinus 16) of the tissue wall 1221. In some examples, the shunt implant 1202 may comprise a barrel portion 1217 configured for placement within the opening 1214 and/or configured to maintain the opening 1214. The first portion 1207 of the sensor implant device 1200 may be configured to pass through and/or fit at least partially within at least a portion of the barrel portion 1217 of the shunt implant 1202. The sensor 1204 may be configured to be situated at any position with respect to the opening 1214 and/or the shunt implant 1202. For example, the sensor 1204 may be configured to be situated above at least a portion of the barrel portion 1217 of the shunt implant 1202. However, the sensor 1204 may additionally or alternatively be situated offset from the barrel portion 1217 of the shunt implant 1202 and/or the opening 1214 of the tissue wall 1221 and/or may extend at least partially over the tissue wall 1221.

In some examples, the shunt body 1203 may be configured to extend through the tissue wall 1221 and/or to position and/or extend the sensor 1204 beyond the opening 1214 and/or the first side 1222 of the tissue wall 1221. For example, the sensor 1204 may be positioned at least partially and/or entirely within the left atrium 2 and/or other chamber on the first side 1222 of the tissue wall 1221. At least a portion of the shunt body 1203 (e.g., the second portion 1209) may additionally or alternatively be configured to extend beyond the second side 1223 of the tissue wall 1221 and/or to anchor within the coronary sinus 16 and/or other blood flow pathway and/or chamber on the second side 1223 of the tissue wall 1221.

The sensor implant device 1200 may be configured for delivery in combination with and/or separately from the shunt implant 1202. For example, the sensor implant device 1200 and the shunt implant 1202 may be delivered during a single delivery procedure. In some examples, the sensor implant device 1200 may be delivered during a first procedure and the shunt implant 1202 may be delivered during a subsequent procedure. Alternatively, the shunt implant 1202 may be delivered during the first procedure and the sensor implant device 1200 may be delivered during a subsequent procedure.

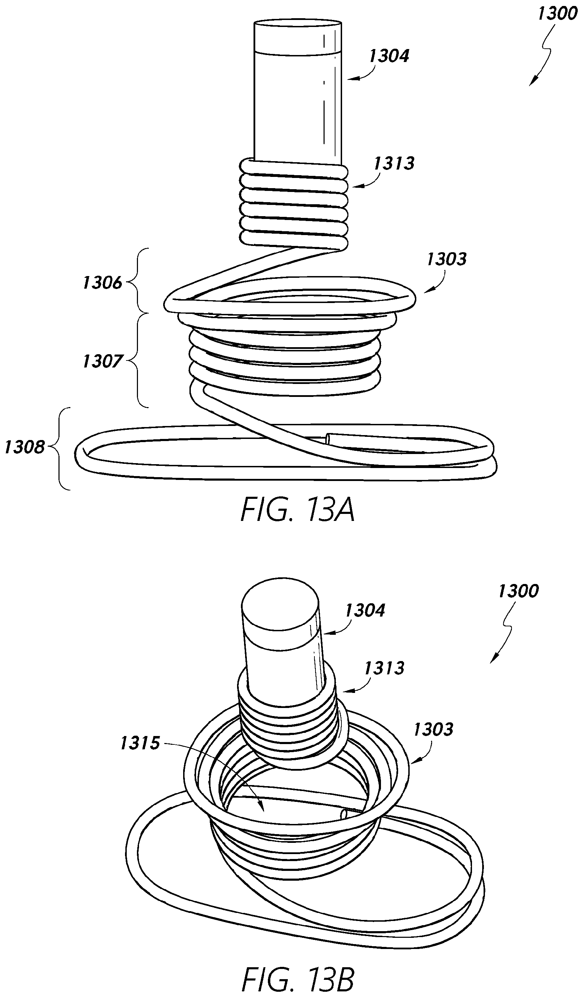

Figures 13A-13D illustrate another sensor implant device 1300 comprising a sensor 1304 coupled to a shunt body 1303 (e.g., a coil and/or coiled wire) configured to secure the sensor 1304 at a desired position within a heart, in accordance with one or more embodiments of the presently claimed invention. The shunt body 1303 may be configured to couple to the sensor 1304 in any of a variety of ways. For example, the shunt body 1303 may be configured to form one or more wraps and/or coils 1313 around the sensor implant to securely attach to the sensor 1304.

The shunt body 1303 may comprise multiple connected devices (e.g., multiple coils and/or coiled wires) or may comprise a single device (e.g., a single coil and/or coiled wire). The shunt body 1303 has a variable shape and/or width and/or diameter. For example, the coil implant may comprise a first portion 1306, a second portion 1307, and/or a third portion 1308. The first portion 1306 may be configured for placement above an opening in a tissue wall 1321 and or may be configured to be situated at least partially in contact with a first side 1322 of the tissue wall 1321. The first portion 1306 of the shunt body 1303 may have a first width and/or diameter. The second portion 1307 of the shunt body 1303 may be configured for placement at least partially within an opening of the tissue wall 1321 and/or may have a second width and/or diameter. The third portion 1308 of the shunt body 1303 may have a third width and/or diameter. The second width and/or diameter may be less than the first width and/or diameter of the first portion 1306 and/or less than the third width and/or diameter of the third portion 1308. The second portion 1307 of the shunt body 1303 may be configured to be situated at least partially within the opening in the tissue wall 1321. The second portion 1307 of the shunt body 1303 may have a generally cylindrical form to approximate a shape of an opening in the tissue wall 1321. The shunt body 1303 may be configured to expand at the third portion 1308 of the shunt body 1303. The third portion 1308 of the shunt body 1303 may be configured to be situated below the opening the tissue wall and/or may be configured to be situated at least partially in contact with a second side 1323 of the tissue wall 1321 (e.g., a coronary sinus 16 side of the tissue wall 1321). The third portion 1308 of the shunt body 1303 may have any suitable shape, for example the third portion 1308 may be oval-shaped, as shown in Figures 13A-13C.

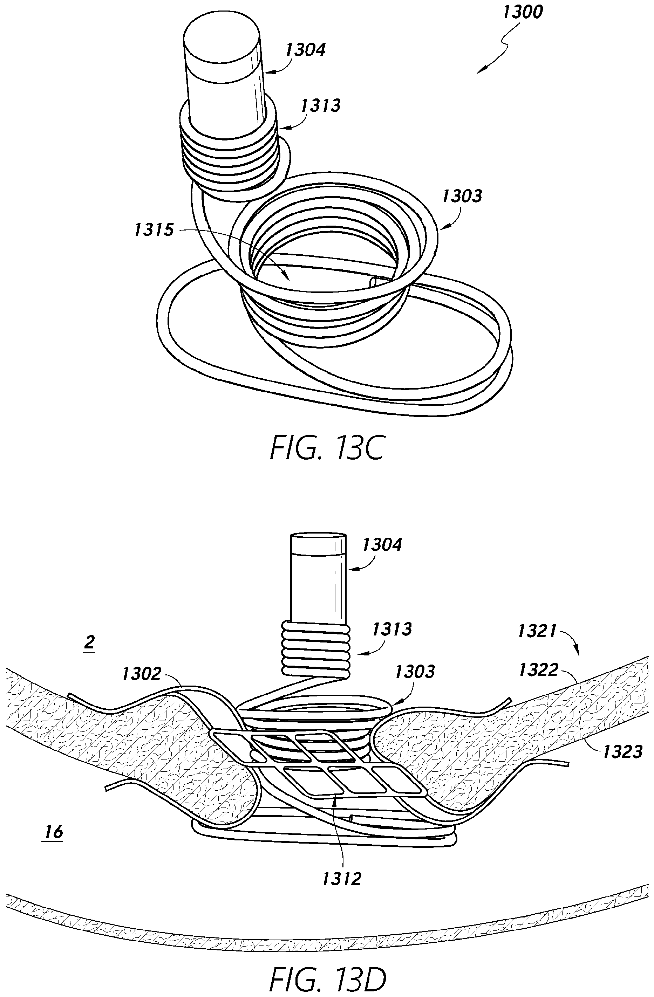

As shown in Figure 13B, the shunt body 1303 may have a generally tubular and/or hollow form around an inner lumen 1315 configured to allow blood flow through the shunt body 1303. In the example shown in Figure 13B, the sensor 1304 may be positioned above the inner lumen 1315. Alternatively, as shown in Figure 13C, the sensor 1304 may be at least partially offset from the inner lumen 1315.

In some examples, the shunt body 1303 may be configured to extend through the tissue wall 1321 and/or to position and/or extend the sensor 1304 beyond the opening 1314 and/or the first side 1322 of the tissue wall 1321. For example, the sensor 1304 may be positioned at least partially and/or entirely within the left atrium 2 and/or other chamber on the first side 1322 of the tissue wall 1321. The shunt body 1303 may additionally or alternatively be configured to extend beyond the second side 1323 of the tissue wall 1321 and/or to anchor within the coronary sinus 16 and/or other blood flow pathway and/or chamber on the second side 1323 of the tissue wall 1321

In some examples, the shunt body 1303 may be at least partially composed of one or more shape-memory alloys. For example, the shunt body 1303 may be configured to be molded to a generally linear form during delivery through a delivery device (e.g., a catheter). As the shunt body 1303 exits the catheter, the shunt body 1303 may be configured to naturally form the device shown in Figures 13A-13D. In some examples, the shunt body 1303 may be configured for delivery subsequent to delivery of a shunt implant 1302.

While the sensor implant device 1300 may be utilized in some cases independently of other implant devices, the sensor implant device 1300 may additionally or alternatively be used in combination with one or more additional implant devices. In the presently claimed invention, as shown for example in Figure 13D, the sensor implant device 1300 is utilized in combination with a shunt implant 1302 which may be configured to form a fluid conduit through the opening in the tissue wall 1321. The shunt implant 1302 may comprise one or more anchoring arms configured to anchor to a first side 1322 and/or to a second side (i.e., the upper wall 1323 of the coronary sinus 16) of the tissue wall 1321. In some examples, the shunt implant 1302 may comprise a barrel portion 1312 configured for placement within the opening 1314 and/or configured to form a fluid conduit through the opening 1314. The second portion 1307 of the sensor implant device 1300 may be configured to pass through at least a portion of the barrel portion 1312 of the shunt implant 1302. The sensor 1304 may be configured to be situated at any position with respect to the opening and/or the shunt implant 1302. For example, the sensor 1304 may be configured to be situated above at least a portion of the barrel portion 1312 of the shunt implant 1302. However, the sensor 1304 may additionally or alternatively be situated offset from the barrel portion 1312 of the shunt implant 1302 and/or the opening of the tissue wall 1321.

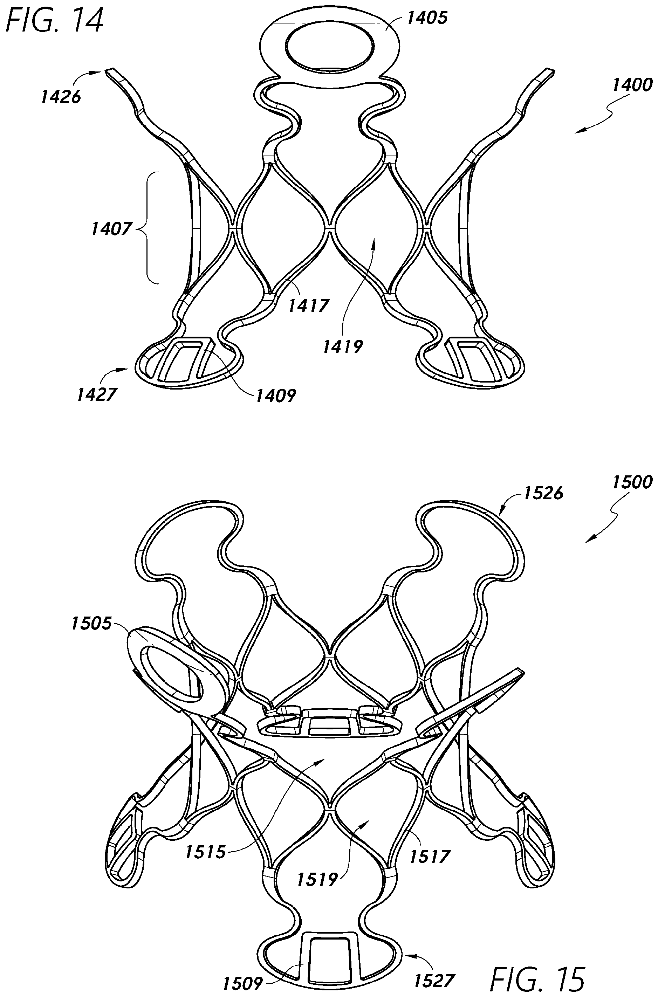

Figures 14-17 illustrate at least portions of a sensor implant devices configured to position one or more sensors at or near an opening of a tissue wall, in accordance with one or more embodiments of the presently claimed invention. Figure 14 provides a side view of an example shunt body 1400 comprising a sensor dock 1405 configured to couple to and/or otherwise mate with one or more sensors. The shunt body 1400 can comprise a network of one or more wires 1417 and/or struts, which can include cords and or similar devices. In some examples, the shunt body 1400 may be configured to form a barrel portion 1407 having a reduced diameter with respect to end portions 1426, 1427 of the shunt body 1400. One or more end portions 1426, 1427 may be configured to extend beyond and/or out of an opening to a tissue wall and/or the barrel portion 1407 may be configured to be positioned at least partially within the opening. The shunt body 1400 has an "hourglass" shape such that the diameter of the shunt body 1400 may gradually decrease between a first end portion 1426 and the barrel portion 1407 and/or may gradually increase from the barrel portion 1407 to a second end portion 1427. A diameter and/or width of the shunt body 1400 may increase from a first (e.g., minimal) diameter and/or width at the barrel portion 1407 to a second (e.g., maximal) diameter and/or width at the first end portion 1426 and/or second end portion 1427 that is greater than the first diameter and/or width.

The shunt body 1400 may have any suitable shape and/or the network of wires forming the shunt body 1400 may have any suitable pattern. In some examples, the shunt body 1400 may be at least partially compressible and/or expandable to allow the shunt body 1400 to assume a compressed form while within a delivery device and/or to expand upon removal from the delivery device. The shunt body 1400 may be configured to be retrievable following delivery of the shunt body 1400. In some examples, the shunt body 1400 may be at least partially composed of shape-memory alloys and/or may otherwise be configured to be shape-set in the form shown in Figure 14 such that the shunt body 1400 may naturally assume the shape shown in Figure 14 following removal from a delivery device.

The shunt body 1400 may comprise one or more cells 1419, which can include gaps and/or openings in the network of wires 1417 forming the shunt body 1400. One or more cells 1419 may have a diamond shape.

In some examples, the shunt body 1400 may be configured to be rotated and or otherwise adjusted following delivery to a desired position with respect to a tissue wall. For example, following placement of at least a portion of the shunt body 1400 within an opening through a tissue wall, the shunt body 1400 may be rotated laterally to adjust a position of the sensor dock 1405 and/or to adjust a position of one or more sensors coupled to the sensor dock 1405. Accordingly, one or more sensors coupled to the shunt body 1400 may be rotationally oriented as desired. In some examples, the sensor dock 1405 may be configured to position one or more sensors at least partially extending over the barrel portion 1407 of the shunt body 1400 and/or extending to an offset position from the barrel portion 1407. The sensor dock 1405 may have a generally circular and/or ring-shaped form as shown in Figure 14. However, the sensor dock 1405 may have any suitable shape and/or may be configured to provide a platform for placement of one or more sensors. In some examples, the sensor dock 1405 may be configured to extend away from the barrel portion 1407 and/or one or more wires 1417 coupled to and/or extending into the sensor dock 1405. The sensor dock 1405 may be configured to extend away from the barrel portion 1407 to prevent occlusion of the barrel portion 1407.

The shunt body 1400 may comprise one or more delivery system attachment features 1409 configured for attachment to various delivery devices. For example, the attachment features 1409 may comprise loops configured to be engaged using hooks of the delivery system.

Figure 15 provides a side view of an example shunt body 1500 comprising a sensor dock 1505 configured to couple to and/or otherwise mate with one or more sensors. The shunt body 1500 can comprise a network of one or more wires 1517, which can include cords and or similar devices. In some examples, the shunt body 1500 may be configured to form a barrel portion having a reduced diameter with respect to end portions 1526, 1527 of the shunt body 1500. One or more end portions 1526, 1527 may be configured to extend beyond and/or out of an opening to a tissue wall and/or the barrel portion may be configured to be positioned at least partially within the opening. The shunt body 1500 has an "hourglass" shape such that the diameter of the shunt body 1500 may gradually decrease between a first end portion 1526 and the barrel portion and/or may gradually increase from the barrel portion to a second end portion 1527. The shunt body 1500 may comprise an inner lumen 1515 through the barrel portion and/or end portions 1526, 1527 of the shunt body 1500.

The shunt body 1500 may have any suitable shape and/or the network of wires forming the shunt body 1500 may have any suitable pattern. In some examples, the shunt body 1500 may be at least partially compressible and/or expandable to allow the shunt body 1500 to assume a compressed form while within a delivery device and/or to expand upon removal from the delivery device. The shunt body 1500 may be configured to be retrievable following delivery of the shunt body 1500. In some examples, the shunt body 1500 may be at least partially composed of shape-memory alloys and/or may otherwise be configured to be shape-set in the form shown in Figure 15 such that the shunt body 1500 may naturally assume the shape shown in Figure 15 following removal from a delivery device.

The shunt body 1500 may comprise one or more cells 1519, which can include gaps and/or openings in the network of wires 1517 forming the shunt body 1500. One or more cells 1519 may have a diamond shape.

In some examples, the shunt body 1500 may be configured to be rotated and or otherwise adjusted following delivery to a desired position with respect to a tissue wall. For example, following placement of at least a portion of the shunt body 1500 within an opening through a tissue wall, the shunt body 1500 may be rotated laterally to adjust a position of the sensor dock 1505 and/or to adjust a position of one or more sensors coupled to the sensor dock 1505. Accordingly, one or more sensors coupled to the shunt body 1500 may be rotationally oriented as desired. In some examples, the sensor dock 1505 may be configured to position one or more sensors at least partially extending over the barrel portion of the shunt body 1500 and/or extending to an offset position from the barrel portion. The sensor dock 1505 may have a generally circular form as shown in Figure 15. However, the sensor dock 1505 may have any suitable shape and/or may be configured to provide a platform for placement of one or more sensors. In some examples, the sensor dock 1505 may be configured to extend away from the barrel portion and/or one or more wires 1517 coupled to and/or extending into the sensor dock 1505. The sensor dock 1505 may be configured to extend away from the barrel portion to prevent occlusion of the barrel portion.

The shunt body 1500 may comprise one or more delivery system attachment features 1509 (e.g., protrusions, hooks, pegs, notches, loops, etc.) configured for attachment to various delivery devices. For example, the attachment features 1509 may comprise loops configured to be engaged using hooks of the delivery system.

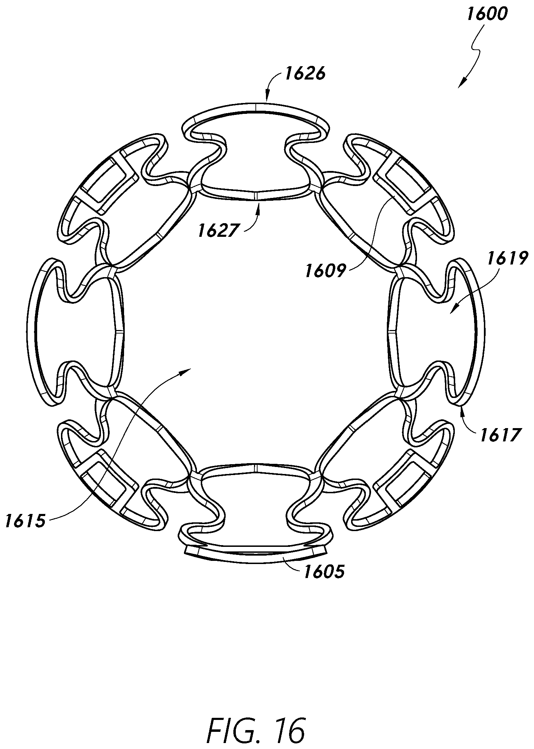

Figure 16 provides an overhead view of an example shunt body 1600 comprising a sensor dock 1605 configured to couple to and/or otherwise mate with one or more sensors. The shunt body 1600 can comprise a network of one or more wires 1617, which can include cords and or similar devices. **In** some examples, the shunt body 1600 may be configured to form a barrel portion having a reduced diameter with respect to end portions 1626, 1627 of the shunt body 1600. One or more end portions 1626, 1627 may be configured to extend beyond and/or out of an opening to a tissue wall and/or the barrel portion may be configured to be positioned at least partially within the opening. The shunt body 1600 has an "hourglass" shape such that the diameter of the shunt body 1600 may gradually decrease between a first end portion 1626 and the barrel portion and/or may gradually increase from the barrel portion to a second end portion 1627. The shunt body 1600 may comprise an inner lumen 1615 through the barrel portion and/or end portions 1626, 1627 of the shunt body 1600.

The shunt body 1600 may have any suitable shape and/or the network of wires forming the shunt body 1600 may have any suitable pattern. In some examples, the shunt body 1600 may be at least partially compressible and/or expandable to allow the shunt body 1600 to assume a compressed form while within a delivery device and/or to expand upon removal from the delivery device. The shunt body 1600 may be configured to be retrievable following delivery of the shunt body 1600. In some examples, the shunt body 1600 may be at least partially composed of shape-memory alloys and/or may otherwise be configured to be shape-set in the form shown in Figure 16 such that the shunt body 1600 may naturally assume the shape shown in Figure 16 following removal from a delivery device.

The shunt body 1600 may comprise one or more cells 1619, which can include gaps and/or openings in the network of wires 1617 forming the shunt body 1600. One or more cells 1619 may have a diamond shape.

In some examples, the shunt body 1600 may be configured to be rotated and or otherwise adjusted following delivery to a desired position with respect to a tissue wall. For example, following placement of at least a portion of the shunt body 1600 within an opening through a tissue wall, the shunt body 1600 may be rotated laterally to adjust a position of the sensor dock 1605 and/or to adjust a position of one or more sensors coupled to the sensor dock 1605. Accordingly, one or more sensors coupled to the shunt body 1600 may be rotationally oriented as desired. In some examples, the sensor dock 1605 may be configured to position one or more sensors at least partially extending over the barrel portion of the shunt body 1600 and/or extending to an offset position from the barrel portion. The sensor dock 1605 may have a generally circular form as shown in Figure 16. However, the sensor dock 1605 may have any suitable shape and/or may be configured to provide a platform for placement of one or more sensors. In some examples, the sensor dock 1605 may be configured to extend away from the barrel portion and/or one or more wires 1617 coupled to and/or extending into the sensor dock 1605. The sensor dock 1605 may be configured to extend away from the barrel portion to prevent occlusion of the barrel portion.

The shunt body 1600 may comprise one or more delivery system attachment features 1609 configured for attachment to various delivery devices. For example, the attachment features 1609 may comprise loops configured to be engaged using hooks of the delivery system.

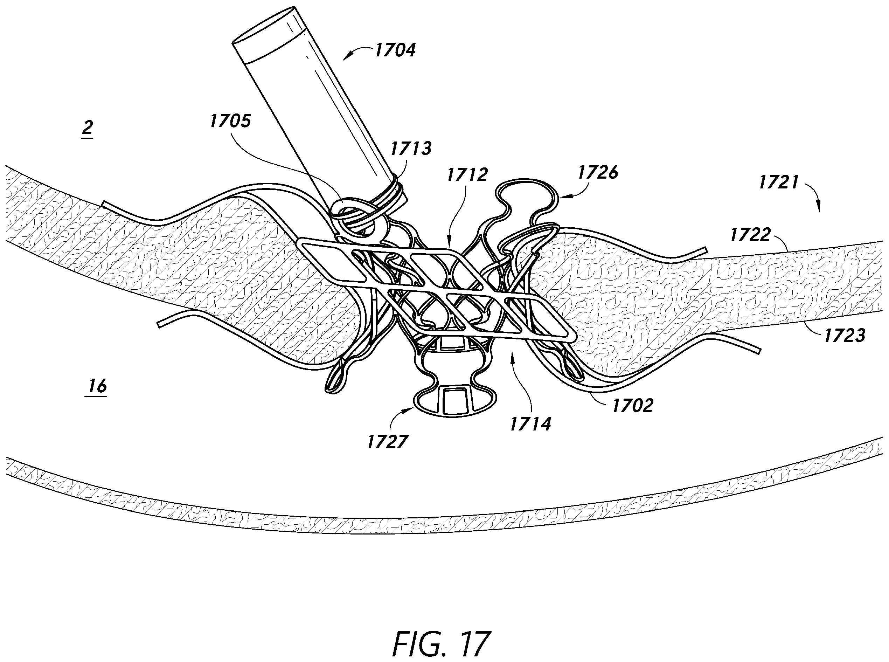

Figure 17 illustrates an example sensor implant system comprising a shunt implant 1702 and a shunt body in accordance with one or more embodiments of the presently claimed invention. The shunt body can comprise a network of one or more wires, which can include cords and or similar devices. In some examples, the shunt body may be configured to form a barrel portion having a reduced diameter with respect to end portions 1726, 1727 of the shunt body. One or more end portions 1726, 1727 may be configured to extend beyond and/or out of an opening 1714 to a tissue wall 1721 and/or the barrel portion may be configured to be positioned at least partially within the opening 1714. The shunt body has an hourglass shape such that the diameter of the shunt body may gradually decrease between a first end portion 1726 and the barrel portion and/or may gradually increase from the barrel portion to a second end portion 1727.

The shunt body may have any suitable shape and/or the network of wires forming the shunt body may have any suitable pattern. In some examples, the shunt body may be at least partially compressible and/or expandable to allow the shunt body to assume a compressed form while within a delivery device and/or to expand upon removal from the delivery device. The shunt body may be configured to be retrievable following delivery of the shunt body. In some examples, the shunt body may be at least partially composed of shape-memory alloys and/or may otherwise be configured to be shape-set in the form shown in Figure 17 such that the shunt body may naturally assume the shape shown in Figure 17 following removal from a delivery device.

In some examples, the shunt body may be configured to be rotated and or otherwise adjusted following delivery to a desired position with respect to a tissue wall 1721. For example, following placement of at least a portion of the shunt body within an opening through a tissue wall, the shunt body may be rotated laterally to adjust a position of the sensor dock 1705 and/or to adjust a position of one or more sensors coupled to the sensor dock 1705. Accordingly, one or more sensors 1704 coupled to the shunt body may be rotationally oriented as desired. In some examples, the sensor dock 1705 may be configured to position one or more sensors at least partially extending over the barrel portion of the shunt body and/or extending to an offset position from the barrel portion. The sensor dock 1705 may have a generally circular form as shown in Figure 17. However, the sensor dock 1705 may have any suitable shape and/or may be configured to provide a platform for placement of one or more sensors. In some examples, the sensor dock 1705 may be configured to extend away from the barrel portion and/or one or more wires coupled to and/or extending into the sensor dock 1705. The sensor dock 1705 may be configured to extend away from the barrel portion to prevent occlusion of the barrel portion. In some examples, one or more coils 1713 may be used to attach the one or more sensors 1704 to the sensor dock 1705.

While the shunt body may be utilized in some cases independently of other implant devices, the shunt body may additionally or alternatively be used in combination with one or more additional implant devices. In the presently claimed invention, the shunt body is utilized in combination with a shunt implant 1702 which may be configured to form a fluid conduit through the opening 1714 in the tissue wall 1721. The shunt implant 1702 may comprise one or more anchoring arms configured to anchor to a first side 1722 and/or to a second side (i.e., the upper wall 1723 of the coronary sinus 16) of the tissue wall 1721. In some examples, the shunt implant 1702 may comprise a barrel portion 1712 configured for placement within the opening 1714 and/or configured to form a fluid conduit through the opening 1714. The barrel portion of the shunt body may be configured to pass through at least a portion of the barrel portion 1712 of the shunt implant 1702. The sensor 1704 may be configured to be situated at any position with respect to the opening 1714 and/or the shunt implant 1702. For example, the sensor 1704 may be configured to be situated above at least a portion of the barrel portion 1712 of the shunt implant 1702. However, the sensor 1704 may additionally or alternatively be situated offset from the barrel portion 1712 of the shunt implant 1702 and/or the opening 1714 of the tissue wall 1721.

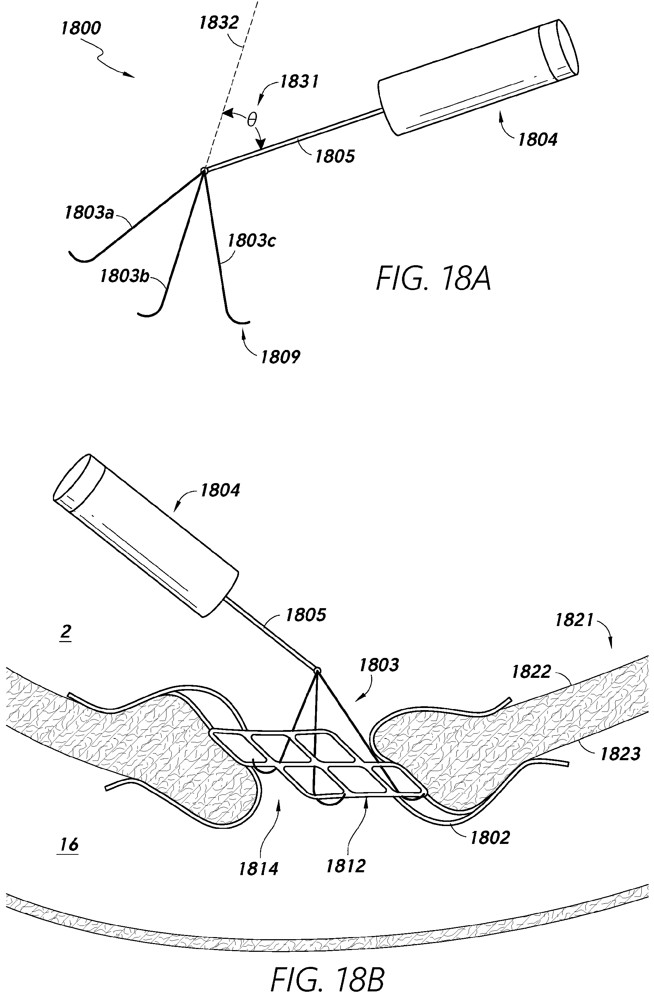

Figures 18A and 18B illustrate a sensor implant device 1800 comprising a sensor 1804 coupled to a shunt body comprising one or more self-expanding anchoring arms 1803 via one or more tethers 1805, in accordance with one or more embodiments of the presently claimed invention. The one or more self-expanding anchoring arms 1803 may comprise attachment features 1809 (e.g., hooked ends) configured to couple to and or otherwise mate with a tissue wall 1821 and/or one or more shunt implant devices 1802. The sensor implant device 1800 is configured for deployment in combination with a shunt implant device 1802, as shown for example in Figure 18B. The one or more anchoring arms 1803 may be configured to hook onto various struts/wires, cells, and/or other features of the shunt implant device 1802. For example, the one or more attachment features 1809 of the sensor implant device 1800 may comprise hooks configured to hook at least partially around one or more struts at a barrel portion 1812 and/or other portion of the shunt implant device 1802. While the sensor implant device 1800 is shown comprising three anchoring arms 1803, the sensor implant device 1800 may comprise any number of anchoring arms 1803.

A tether 1805 coupling the one or more anchoring arms 1803 to the sensor 1804 may have a rigid or flexible structure. In some examples, the tether 1805 may be configured to maintain a given angle 1831 with respect to an axis 1832 of the one or more anchoring arms 1803. For example, the axis 1832 may represent an imaginary line through an opening 1814 of a tissue wall 1821 and/or through a barrel portion 1812 of the shunt implant 1802. The tether 1805 may be configured to position the sensor 1804 outside of and/or offset from an opening 1814 through a tissue wall 1821 and/or a barrel portion 1812 of the shunt implant device 1802.

The shunt implant 1802 may be configured to form a fluid conduit through the opening 1814 in the tissue wall 1821. The shunt implant 1802 may comprise one or more anchoring arms configured to anchor to a first side 1822 and/or to a second side (i.e., the upper wall 1823 of the coronary sinus 16) of the tissue wall 1821. In some examples, the shunt implant 1802 may comprise a barrel portion 1812 configured for placement within the opening 1814 and/or configured to form a fluid conduit through the opening 1814. The one or more anchoring arms 1803 of the sensor implant device 1800 may be configured to pass through at least a portion of the barrel portion 1812 of the shunt implant 1802. The sensor 1804 may be configured to be situated at any position with respect to the opening 1814 and/or the shunt implant 1802. For example, the sensor 1804 may be configured to be situated above at least a portion of the barrel portion 1812 of the shunt implant 1802. However, the sensor 1804 may additionally or alternatively be situated offset from the barrel portion 1812 of the shunt implant 1802 and/or the opening 1814 of the tissue wall 1821.

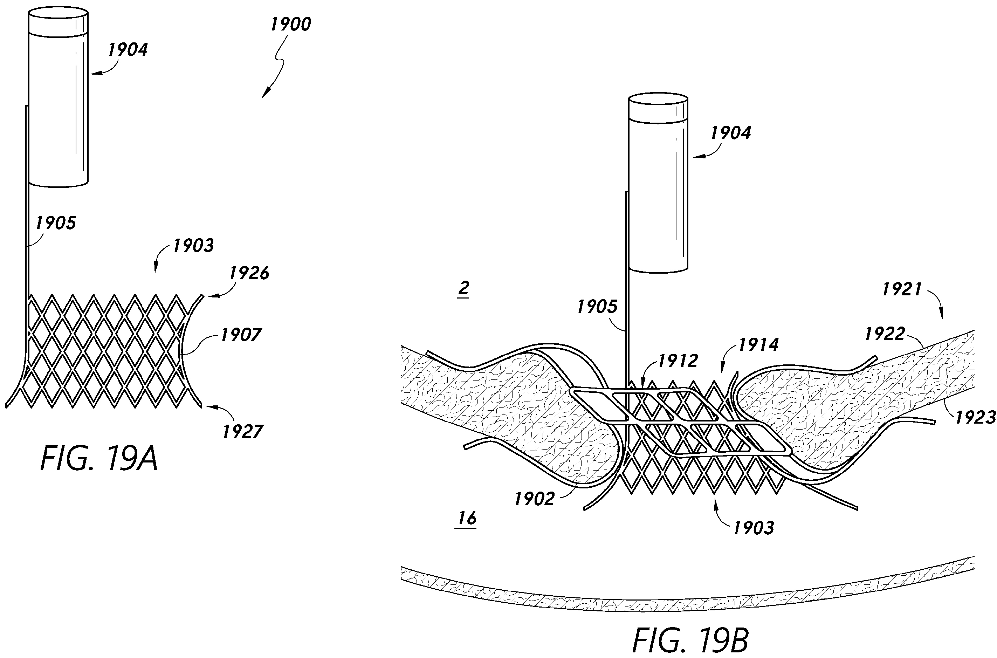

Figures 19A illustrates a sensor implant device 1900 comprising a sensor 1904 coupled to a shunt body 1903 via one or more tethers 1905, in accordance with one or more examples. Figure 19B illustrates a sensor implant system comprising the sensor implant device 1900 and/or a shunt implant device 1902 implanted at an opening 1914 of a tissue wall 1921. The shunt body 1903 may have a generally tubular form and/or may have an hourglass shape. For example, the first implant may have a generally curved form in which a barrel portion 1907 (i.e., midsection) of the shunt body 1903 has a smaller diameter than the shunt body 1903 at a first end 1926 and/or a second end 1927 of the shunt body 1903. **In** some examples, the sensor implant device 1900 may be configured for deployment in combination with a shunt implant device 1902, as shown in Figure 19B. The first end 1926 and/or second end 1927 may be configured to extend at least partially over at least a portion of the shunt implant device 1902 and/or a tissue wall 1921. For example, the first end 1926 may be configured to extend over a first side 1922 of the tissue wall 1921 and/or the second end 1927 may be configured to extend over a second side 1923 of the tissue wall 1921.

A tether 1905 coupling the shunt body 1903 to the sensor 1904 may have a rigid or flexible structure. The shunt implant 1902 may be configured to form and/or maintain a fluid conduit through the opening 1914 in the tissue wall 1921. The shunt implant 1902 may comprise one or more anchoring arms configured to anchor to a first side 1922 and/or to a second side (i.e., the upper wall 1923 of the coronary sinus 16) of the tissue wall 1921. In some examples, the shunt implant 1902 may comprise a barrel portion 1912 configured for placement within the opening 1914 and/or configured to form a fluid conduit through the opening 1914. The shunt body 1903 may be configured to pass through at least a portion of the barrel portion 1912 of the shunt implant 1902. The sensor 1904 may be configured to be situated at any position with respect to the opening 1914 and/or the shunt implant 1902. For example, the sensor 1904 may be configured to be situated above at least a portion of the barrel portion 1912 of the shunt implant 1902. However, the sensor 1904 may additionally or alternatively be situated offset from the barrel portion 1912 of the shunt implant 1902 and/or the opening 1914 of the tissue wall 1921.

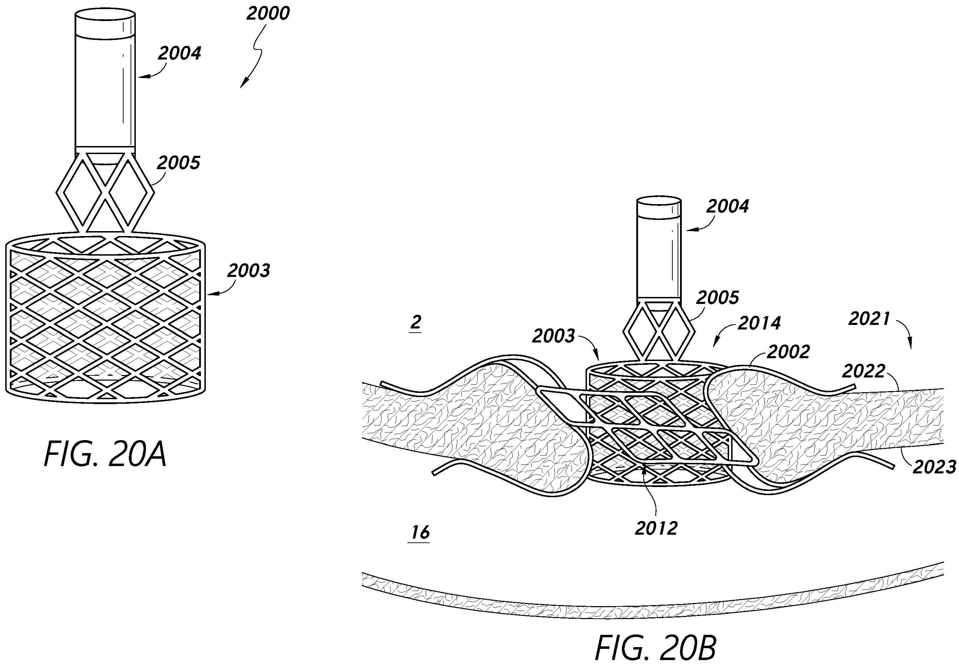

Figures 20A illustrates a sensor implant device 2000 comprising a sensor 2004 coupled to a shunt body 2003 via one or more coupling arms 2005, in accordance with one or more examples. Figure 20B illustrates a sensor implant system comprising the sensor implant device 2000 and/or a shunt implant 2002 implanted at an opening 2014 of a tissue wall 2021. The shunt body 2003 may have a generally tubular form and/or may have an hourglass shape. In some examples, the sensor implant device 2000 may be configured for deployment in combination with a shunt implant device 2002, as shown in Figure 20B. In some examples, the one or more coupling arms 2005 may comprise multiple wires and/or lines forming one or more diamond-shaped and/or triangular cells. For example, the coupling arms 2005 may comprise a network of wires forming two diamond-shaped cells and/or two triangular cells.

One or more coupling arms 2005 coupling the shunt body 2003 to the sensor 2004 may have a rigid or flexible structure. The shunt implant 2002 may be configured to form and/or maintain a fluid conduit through the opening 2014 in the tissue wall 2021. The shunt implant 2002 may comprise one or more anchoring arms configured to anchor to a first side 2022 and/or to a second side (i.e., the upper wall 2023 of the coronary sinus 16) of the tissue wall 2021. In some examples, the shunt implant 2002 may comprise a barrel portion 2012 configured for placement within the opening 2014 and/or configured to form a fluid conduit through the opening 2014. The shunt body 2003 may be configured to pass through at least a portion of the barrel portion 2012 of the shunt implant 2002. The sensor 2004 may be configured to be situated at any position with respect to the opening 2014 and/or the shunt implant 2002. For example, the sensor 2004 may be configured to be situated above at least a portion of the barrel portion 2012 of the shunt implant 2002. However, the sensor 2004 may additionally or alternatively be situated offset from the barrel portion 2012 of the shunt implant 2002 and/or the opening 2014 of the tissue wall 2021.

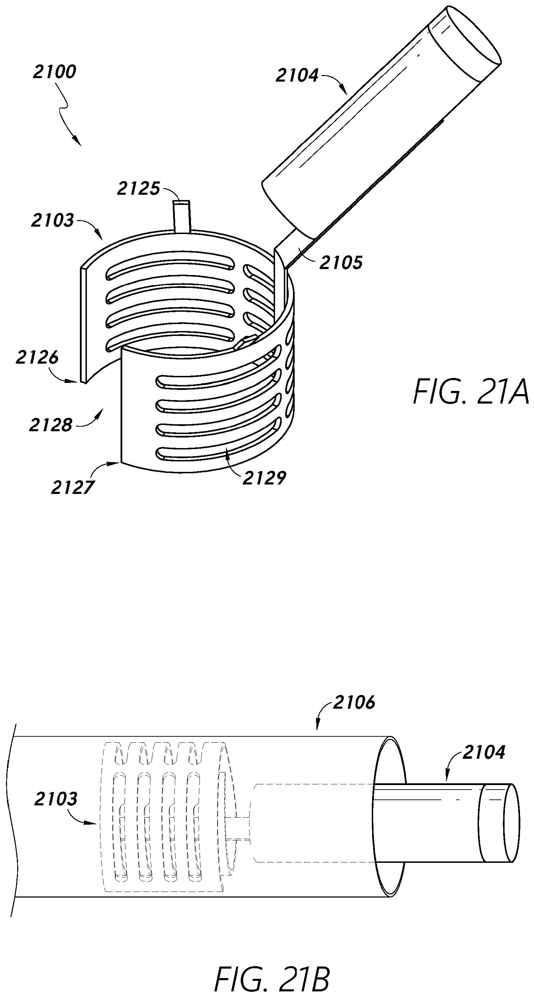

Figures 21A-21C illustrate another sensor implant device 2100 comprising a sensor 2104 coupled to a shunt body 2103 of the sensor implant device 2100, in accordance with one or more embodiments of the presently claimed invention. The shunt body 2103 has a partial cylindrical form with a gap 2128 separating a first end 2126 and a second end 2127 of the shunt body 2103. The shunt body 2103 may comprise one or more elongate apertures 2129 to allow blood flow through the shunt body 2103.

In some examples, the sensor 2104 may be coupled to the shunt body 2103 through use of a sensor dock 2105 and/or arm extending from and/or coupled to the shunt body 2103. The sensor dock 2105 may be configured to position the sensor 2104 at least partially over the shunt body 2103 and/or an opening 2114 through a tissue wall 2121 and/or may be configured to position the sensor 2104 at an angle with respect to the shunt body 2103 and/or at least partially offset from the opening 2114 through the tissue wall 2121.

The shunt body 2103 may comprise one or more tabs 2125 extending from the shunt body 2103. In some examples, the one or more tabs 2125 may be configured for attachment to various delivery systems.

As shown in Figure 21B, the shunt body 2103 is at least partially compressible and/or expandable. The shunt body 2103 is configured to reduce in diameter by allowing the first end 2126 and the second end 2127 to at least partially overlap. In some examples, the sensor 2104 may assume a linear orientation with respect to the shunt body 2103 while within a delivery device 2106 (e.g., a catheter) and/or may be configured to assume an angled configuration with respect to the shunt body 2103 after removal from the delivery device 2106.

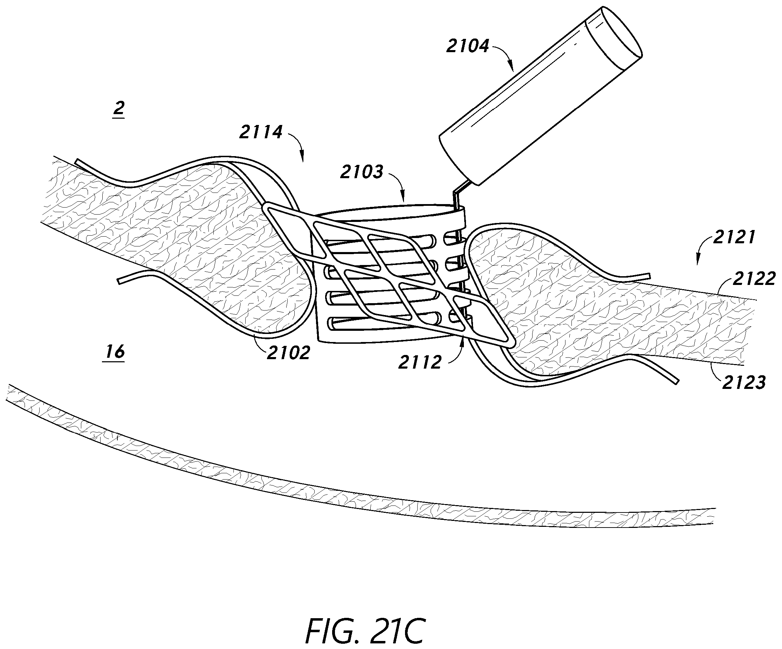

The sensor implant device 2100 is configured for deployment in combination with a shunt implant device 2102, for example as shown in Figure 21C. The shunt implant 2102 may be configured to form and/or maintain a fluid conduit through the opening 2114 in the tissue wall 2121. The shunt implant 2102 may comprise one or more anchoring arms configured to anchor to a first side 2122 and/or to a second side (i.e., the upper wall 2123 of the coronary sinus 16) of the tissue wall 2121. In some examples, the shunt implant 2102 may comprise a barrel portion 2112 configured for placement within the opening 2114 and/or configured to form a fluid conduit through the opening 2114. The shunt body 2103 may be configured to pass through at least a portion of the barrel portion 2112 of the shunt implant 2102. The sensor 2104 may be configured to be situated at any position with respect to the opening 2114 and/or the shunt implant 2102. For example, the sensor 2104 may be configured to be situated above at least a portion of the barrel portion 2112 of the shunt implant 2102. However, the sensor 2104 may additionally or alternatively be situated offset from the barrel portion 2112 of the shunt implant 2102 and/or the opening 2114 of the tissue wall 2121.

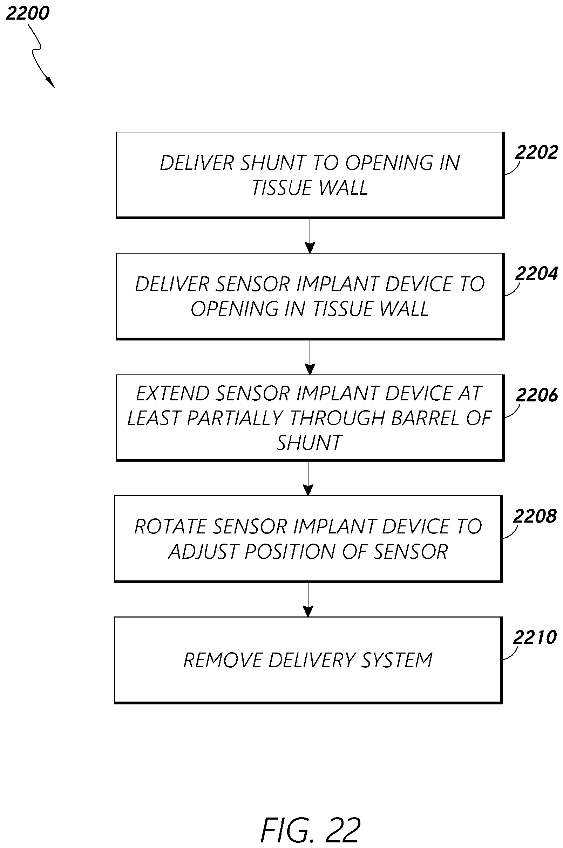

Figure 22 provides a flowchart illustrating steps of a process 2200 for delivering the one or more sensor implant devices and/or shunt devices described herein, in accordance with one or more examples. While the process 2200 is illustrated in a step-by-step order, some steps of the process 2200 may be performed simultaneously and/or in different orders.

At step 2202, the process 2200 involves delivering a shunt implant to an opening in a tissue wall percutaneously and/or via a catheter and/or other delivery systems. The shunt implant can include, for example, the shunt/anchor structure 150 illustrated in Figure 7. For example, the shunt implant can comprise a barrel portion configured for placement at least partially within the opening and/or the shunt implant can comprise one or more anchoring arms configured to anchor to the tissue wall. The shunt implant may be configured to form and/or maintain a fluid conduit through the opening in the tissue wall. The opening may be created during a puncture procedure performed prior to delivery of the shunt implant.

At step 2204, the process 2200 involves delivering a sensor implant device to the opening in the tissue wall percutaneously and/or via a catheter and/or other delivery systems. In some examples, the sensor implant device may be delivered together with the shunt implant and/or may be delivered via the same catheter used to deliver the shunt implant. However, the shunt implant and the sensor implant device may be delivered separately and/or during separate medical procedures. In some examples, the shunt implant may be delivered prior to the sensor implant device. For example, the shunt implant may be anchored at the opening and the sensor implant device may be subsequently delivered and/or may be passed at least partially through the barrel portion of the shunt implant. The sensor implant device may comprise various features, which can include anchoring arms, hooks, expandable shunt bodies, and/or curved ends, configured to anchor to the shunt implant and/or the tissue wall. Alternatively, the sensor implant device may be delivered prior to delivery of the shunt implant. For example, the sensor implant device may comprise various features, which can include anchoring arms, hooks, expandable barrel portions, and/or curved ends, configured to anchor the sensor implant device independently to the tissue wall and/or at least partially within the opening. The shunt implant may subsequently be delivered and/or may pass at least partially through a shunt body of the sensor implant device.

At step 2206, the process 2200 involves extending at least a portion of the sensor implant device at least partially through a barrel portion of the shunt device. For example, the shunt implant may comprise a generally tubular barrel portion forming an inner lumen through the shunt implant. In some examples, the sensor implant device may comprise a generally tubular shunt body that may be smaller in diameter than the barrel portion of the shunt implant such that the shunt body of the sensor implant device may be configured to fit at least partially within the barrel portion of the shunt implant.

Alternatively, the shunt implant may be extended at least partially through the shunt body of the sensor implant device. For example, the barrel portion of the shunt implant may be smaller in diameter than the barrel portion of the sensor implant device such that the barrel portion of the shunt implant may be configured to fit at least partially within the shunt body of the sensor implant device.

At step 2208, the process 2200 involves rotating the sensor implant device as desired to adjust the position of a sensor coupled to the sensor implant device until a desired position of the sensor is achieved. The sensor may extend above, diagonally away from, and/or perpendicularly to the shunt body of the sensor implant device. As the sensor implant device is rotated, the sensor may be moved to be positioned above and/or near different portions of the tissue wall and/or within different portions of a heart chamber.

At step 2210, the process 2200 involves removing the catheter and/or other delivery systems from the body. The shunt and/or sensor implant device may remain within the body.

Some implementations of the present disclosure relate to a sensor implant system comprising a shunt implant comprising a central flow portion configured to maintain an opening through a tissue wall and a sensor implant device comprising a shunt body configured to fit at least partially within the central flow portion of the shunt implant and a sensor coupled to the shunt body.

The sensor may be configured to extend at least partially over the opening through the tissue wall. In some examples, the sensor is configured to extend at least partially over the tissue wall.

In some examples, the sensor is coupled to the shunt body via an arm extending from the shunt body. The arm may form one or more coils around the sensor.

The shunt implant may further comprise one or more anchoring arms. In some examples, the shunt body has a cylindrical shape.

In some examples, the shunt body comprises a wire loop having a proximal portion configured to assume a first width within the central flow portion of the shunt implant and having a distal portion configured to expand to a second width beyond the central flow portion of the shunt implant. The second width may be greater than the first width.

The wire loop may be configured to expand to the second width beyond a first side of the tissue wall. In some examples, the sensor is configured to extend beyond a second side of the tissue wall.

In some examples, the wire loop is at least partially composed of one or more shape-memory alloys. The proximal portion of the wire loop is configured to be situated at least partially within the central flow portion of the shunt implant.

The wire loop may comprise a first end and a second end coupled to the sensor. In some examples, the wire loop is configured to form one or more coils around the sensor.

In some examples, the shunt body comprises a coil having a proximal portion configured to form a first width within the central flow portion of the shunt implant and having a distal portion configured to form a second width beyond the central flow portion of the shunt implant. The second width may be greater than the first width.

The shunt body may be configured to form the second width beyond a first side of the tissue wall. In some examples, the sensor is configured to extend beyond a second side of the tissue wall.

In some examples, the coil comprises a first end configured to form one or more winds around the sensor. The coil may comprise a second end at the distal portion.

The coil may form multiple winds at the proximal portion and at the distal portion. In some examples, the coil is configured to hold the sensor above the proximal portion of the coil.

In some examples, the coil is configured to hold the sensor at least partially offset from the proximal portion of the coil.

The shunt body may have an hourglass shape in which a midsection of the shunt body has a first diameter and the shunt body expands to a second diameter at a first end of the shunt body and at a second end of the shunt body. In some examples, the second diameter is greater than the first diameter.

In some examples, the shunt body comprises a sensor dock at the first end. The sensor may be configured to couple to the shunt body at the sensor dock.

The shunt body may comprise a network of struts forming cells. In some examples, at least a portion of the cells are diamond shaped.

In some examples, the shunt body comprises one or more protrusions at the second end configured to mate with one or more delivery devices. The shunt body may have a partial cylindrical form with a gap separating a first end of the shunt body from a second end of the shunt body.

The shunt body may be configured to assume a compressed form during delivery, in which the first end at least partially overlaps with the second end.

In some examples, the shunt body comprises multiple anchoring arms configured to mate with the shunt implant. Each of the anchoring arms may couple to a tether interconnecting the sensor and the anchoring arms.

The tether may be configured to extend the sensor at least partially over the tissue wall. In some examples, the sensor implant system further comprises a coupling arm interconnecting the sensor and the shunt body. The coupling arm may comprise a network of struts forming diamond shaped cells.

Some implementations of the present disclosure relate to a method comprising delivering a shunt implant to an opening in a tissue wall. The shunt implant comprises a central flow portion configured to maintain the opening. The method further comprises delivering a sensor implant device to the opening in the tissue wall. The sensor implant device comprises a shunt body configured to fit at least partially within the central flow portion of the shunt implant and a sensor coupled to the shunt body. The method further comprises rotating the sensor implant device to adjust a position of the sensor.

In some examples, the shunt body comprises a coil configured to form a first width within the central flow portion of the shunt implant and configured to form a second width beyond the central flow portion of the shunt implant. The second width may be greater than the first width.

The shunt body may be configured to form the second width beyond a first side of the tissue wall. In some examples, the sensor is configured to extend beyond a second side of the tissue wall.

In accordance with some implementations of the present disclosure, a sensor implant device comprises a shunt body configured to fit at least partially within a central flow portion of a shunt implant and a sensor coupled to the shunt body.

The shunt body may have an hourglass shape in which a midsection of the shunt body has a first diameter and the shunt body expands to a second diameter at a first end of the shunt body and at a second end of the shunt body. In some examples, the second diameter is greater than the first diameter.

In some examples, the shunt body comprises a sensor dock at the first end. The sensor may be configured to couple to the shunt body at the sensor dock.

The shunt body may comprise a network of struts forming cells. In some examples, at least a portion of the cells are diamond shaped.

In some examples, the shunt body comprises one or more protrusions at the second end configured to mate with one or more delivery devices. The sensor may be configured to extend at least partially over the opening through the tissue wall.

The sensor may be configured to extend at least partially over the tissue wall. In some examples, the sensor is coupled to the shunt body via an arm extending from the shunt body.

In some examples, the arm forms one or more coils around the sensor. The shunt body may have a cylindrical shape.

The shunt body may comprise a wire loop having a proximal portion configured to assume a first width within an opening of a tissue wall and having a distal portion configured to expand to a second width beyond the opening of the tissue wall. In some examples, the second width is greater than the first width.

In some examples, the wire loop is configured to expand to the second width beyond a first side of the tissue wall. The sensor may be configured to extend beyond a second side of the tissue wall.

The wire loop may be at least partially composed of one or more shape-memory alloys. In some examples, the wire loop comprises a first end and a second end coupled to the sensor.

In some examples, the wire loop is configured to form one or more coils around the sensor.

The shunt body may comprise a coil having a proximal portion configured to form a first width within an opening of a tissue wall and having a distal portion configured to form a second width beyond the opening of the tissue wall. In some examples, the second width is greater than the first width.

In some examples, the shunt body is configured to form the second width beyond a first side of the tissue wall. The sensor may be configured to extend beyond a second side of the tissue wall.

The coil may comprise a first end configured to form one or more winds around the sensor. In some examples, the coil comprises a second end at the distal portion.

In some examples, the coil forms multiple winds at the proximal portion and at the distal portion. The coil may be configured to hold the sensor above the proximal portion of the coil.

The coil may be configured to hold the sensor at least partially offset from the proximal portion of the coil. In some examples, the shunt body has a partial cylindrical form with a gap separating a first end of the shunt body from a second end of the shunt body.

In some examples, the shunt body is configured to assume a compressed form during delivery in which the first end at least partially overlaps with the second end.

The sensor implant device may further comprise a coupling arm interconnecting the sensor and the shunt body. In some examples, the coupling arm comprises a network of struts forming diamond shaped cells.

### Additional Examples

Depending on the example, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the inventions herein disclosed and claimed below should not be limited by the particular examples described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A sensor implant system comprising:
a shunt implant (1202) comprising a central flow portion configured to maintain an opening (1214) through a tissue wall (1221); and
a sensor implant device (1200) including a shunt body (1203) configured to fit at least partially within the central flow portion of the shunt implant and a sensor (1204) coupled to the shunt body;
wherein the shunt body comprises a wire loop having a proximal portion configured to assume a first width within the central flow portion of the shunt implant and having a distal portion configured to expand to a second width beyond the central flow portion of the shunt implant, and wherein the second width is greater than the first width.

2. The sensor implant system of claim 1, wherein the wire loop is:
a) configured to expand to the second width beyond a first side (1223) of the tissue wall, and wherein the sensor is configured to extend beyond a second side (1222) of the tissue wall; and/or
b) at least partially composed of one or more shape-memory alloys.

3. The sensor implant system of claim 1 or claim 2, wherein the proximal portion of the wire loop is configured to be situated at least partially within the central flow portion of the shunt implant.

4. The sensor implant system of any of claims 1 to 3, wherein the wire loop:
a) comprises a first end (1226) and a second end (1227) coupled to the sensor; and/or
b) is configured to form one or more coils around the sensor.

5. A sensor implant system comprising:
a shunt implant (1302) comprising a central flow portion configured to maintain an opening (1314) through a tissue wall (1321); and
a sensor implant device (1300) including a shunt body (1303) configured to fit at least partially within the central flow portion of the shunt implant and a sensor (1304) coupled to the shunt body;
wherein the shunt body comprises a coil having a proximal portion (1307) configured to form a first width within the central flow portion of the shunt implant and having a distal portion (1308) configured to form a second width beyond the central flow portion of the shunt implant, and wherein the second width is greater than the first width.

6. The sensor implant system of claim 5, wherein the shunt body is configured to form the second width beyond a first side (1323) of the tissue wall, and wherein the sensor is configured to extend beyond a second side (1322) of the tissue wall.

7. The sensor implant system of claim 5 or claim 6, wherein the coil:
a) comprises a first end configured to form one or more winds (1313) around the sensor;
b) comprises a second end at the distal portion; and/or
c) forms multiple winds at the proximal portion and at the distal portion.

8. The sensor implant system of any of claims 5 to 7, wherein the coil is configured to hold the sensor:
a) above the proximal portion of the coil; and/or
b) at least partially offset from the proximal portion of the coil.

9. A sensor implant system comprising:
a shunt implant (1702) comprising a central flow portion configured to maintain an opening (1714) through a tissue wall (1721); and
a sensor implant device including a shunt body (1400) configured to fit at least partially within the central flow portion of the shunt implant and a sensor (1704) coupled to the shunt body;
wherein the shunt body has an hourglass shape in which a midsection (1407) of the shunt body has a first diameter and the shunt body expands to a second diameter at a first end (1426) of the shunt body and at a second end (1427) of the shunt body, wherein the second diameter is greater than the first diameter.

10. The sensor implant system of claim 9, wherein the shunt body comprises:
a) a sensor dock (1405) at the first end, and wherein the sensor is configured to couple to the shunt body at the sensor dock;
b) a network of struts (1417) forming cells (1419), optionally wherein at least a portion of the cells are diamond shaped; and/or
c) one or more protrusions (1409) at the second end configured to mate with one or more delivery devices.

11. A sensor implant system comprising:
a shunt implant (2102) comprising a central flow portion configured to maintain an opening (2114) through a tissue wall (2121); and
a sensor implant device including a shunt body (2100) configured to fit at least partially within the central flow portion of the shunt implant and a sensor (2104) coupled to the shunt body;
wherein the shunt body has a partial cylindrical form with a gap (2128) separating a first end (2126) of the shunt body from a second end (2127) of the shunt body; and
wherein the shunt body is configured to assume a compressed form during delivery, in which the first end at least partially overlaps with the second end.

12. A sensor implant system comprising:
a shunt implant (1802) comprising a central flow portion configured to maintain an opening (1814) through a tissue wall (1821); and
a sensor implant device including a shunt body (1800) configured to fit at least partially within the central flow portion of the shunt implant and a sensor (1804) coupled to the shunt body;
wherein the shunt body comprises multiple anchoring arms (1803) configured to mate with the shunt implant,
wherein each of the anchoring arms couples to a tether (1805) interconnecting the sensor and the anchoring arms; and
wherein the tether is configured to extend the sensor at least partially over the tissue wall.

13. The sensor implant system of any of claims 1 to 12, wherein the sensor is configured to extend:
a) at least partially over the opening through the tissue wall; and/or
b) at least partially over the tissue wall.

14. The sensor implant system of any of claims 1 to 13, wherein the shunt implant further comprises one or more anchoring arms (152, 154).

## Patentansprüche

1. Sensorimplantatsystem, das Folgendes umfasst:
ein Shuntimplantat (1202), das einen zentralen Strömungsabschnitt umfasst, der dazu ausgelegt ist, eine Öffnung (1214) durch eine Gewebewand (1221) aufrechtzuerhalten; und
eine Sensorimplantatvorrichtung (1200), die einen Shuntkörper (1203), der dazu ausgelegt ist, zumindest teilweise in den zentralen Strömungsabschnitt des Shuntimplantats zu passen, und einen mit dem Shuntkörper gekoppelten Sensor (1204) umfasst;
wobei der Shuntkörper eine Drahtschlaufe umfasst, die einen proximalen Abschnitt aufweist, der dazu ausgelegt ist, eine erste Breite innerhalb des zentralen Strömungsabschnitts des Shuntimplantats anzunehmen, und einen distalen Abschnitt aufweist, der dazu ausgelegt ist, sich auf eine zweite Breite über den zentralen Strömungsabschnitt des Shuntimplantats hinaus zu erweitern, und wobei die zweite Breite größer als die erste Breite ist.

2. Sensorimplantatsystem nach Anspruch 1, wobei die Drahtschlaufe:
a) dazu ausgelegt ist, sich auf die zweite Breite über eine erste Seite (1223) der Gewebewand hinaus zu erweitern, und wobei der Sensor dazu ausgelegt ist, sich über eine zweite Seite (1222) der Gewebewand hinaus auszudehnen; und/oder
b) zumindest teilweise aus einer oder mehreren Formgedächtnislegierungen besteht.

3. Sensorimplantatsystem nach Anspruch 1 oder Anspruch 2, wobei der proximale Abschnitt der Drahtschlaufe dazu ausgelegt ist, zumindest teilweise innerhalb des zentralen Strömungsabschnitts des Shuntimplantats angeordnet zu sein.

4. Sensorimplantatsystem nach einem der Ansprüche 1 bis 3, wobei die Drahtschlaufe:
a) ein erstes Ende (1226) und ein mit dem Sensor gekoppeltes zweites Ende (1227) umfasst; und/oder
b) dazu ausgelegt ist, eine oder mehrere Spulen um den Sensor herum zu bilden.

5. Sensorimplantatsystem, das Folgendes umfasst:
ein Shuntimplantat (1302), das einen zentralen Strömungsabschnitt umfasst, der dazu ausgelegt ist, eine Öffnung (1314) durch eine Gewebewand (1321) aufrechtzuerhalten; und
eine Sensorimplantatvorrichtung (1300), die einen Shuntkörper (1303), der dazu ausgelegt ist, zumindest teilweise in den zentralen Strömungsabschnitt des Shuntimplantats zu passen, und einen mit dem Shuntkörper gekoppelten Sensor (1304) umfasst;
wobei der Shuntkörper eine Spule umfasst, die einen proximalen Abschnitt (1307) aufweist, der dazu ausgelegt ist, eine erste Breite innerhalb des zentralen Strömungsabschnitts des Shuntimplantats zu bilden, und einen distalen Abschnitt (1308) aufweist, der dazu ausgelegt ist, eine zweite Breite über den zentralen Strömungsabschnitt des Shuntimplantats hinaus zu bilden, und wobei die zweite Breite größer als die erste Breite ist.

6. Sensorimplantatsystem nach Anspruch 5, wobei der Shuntkörper dazu ausgelegt ist, die zweite Breite über eine erste Seite (1323) der Gewebewand hinaus zu bilden, und wobei der Sensor dazu ausgelegt ist, sich über eine zweite Seite (1322) der Gewebewand hinaus auszudehnen.

7. Sensorimplantatsystem nach Anspruch 5 oder Anspruch 6, wobei die Spule:
a) ein erstes Ende umfasst, das dazu ausgelegt ist, einen oder mehrere Wicklungen (1313) um den Sensor herum zu bilden;
b) ein zweites Ende an dem distalen Abschnitt umfasst; und/oder
c) mehrere Wicklungen am proximalen Abschnitt und am distalen Abschnitt bildet.

8. Sensorimplantatsystem nach einem der Ansprüche 5 bis 7, wobei die Spule dazu ausgelegt ist, den Sensor folgendermaßen zu halten:
a) über dem proximalen Abschnitt der Spule; und/oder
b) zumindest teilweise versetzt von dem proximalen Abschnitt der Spule.

9. Sensorimplantatsystem, das Folgendes umfasst:
ein Shuntimplantat (1702), das einen zentralen Strömungsabschnitt umfasst, der dazu ausgelegt ist, eine Öffnung (1714) durch eine Gewebewand (1721) aufrechtzuerhalten; und
eine Sensorimplantatvorrichtung, die einen Shuntkörper (1400), der dazu ausgelegt ist, zumindest teilweise in den zentralen Strömungsabschnitt des Shuntimplantats zu passen, und einen mit dem Shuntkörper gekoppelten Sensor (1704) umfasst;
wobei der Shuntkörper eine Sanduhrform aufweist, bei der ein Mittelabschnitt (1407) des Shuntkörpers einen ersten Durchmesser aufweist und sich der Shuntkörper an einem ersten Ende (1426) des Shuntkörpers und an einem zweiten Ende (1427) des Shuntkörpers auf einen zweiten Durchmesser erweitert, wobei der zweite Durchmesser größer als der erste Durchmesser ist.

10. Sensorimplantatsystem nach Anspruch 9, wobei der Shuntkörper Folgendes umfasst:
a) eine Sensorandockvorrichtung (1405) am ersten Ende, und wobei der Sensor dazu ausgelegt ist, an der Sensorandockvorrichtung mit dem Shuntkörper gekoppelt zu sein;
b) ein Netzwerk aus Streben (1417), die Zellen (1419) bilden, wobei optional zumindest ein Teil der Zellen rautenförmig ist; und/oder
c) einen oder mehrere Vorsprünge (1409) am zweiten Ende, die dazu ausgelegt sind, mit einer oder mehreren Einführungsvorrichtungen zusammenzupassen.

11. Sensorimplantatsystem, das Folgendes umfasst:
ein Shuntimplantat (2102), das einen zentralen Strömungsabschnitt umfasst, der dazu ausgelegt ist, eine Öffnung (2114) durch eine Gewebewand (2121) aufrechtzuerhalten; und
eine Sensorimplantatvorrichtung, die einen Shuntkörper (2100), der dazu ausgelegt ist, zumindest teilweise in den zentralen Strömungsabschnitt des Shuntimplantats zu passen, und einen mit dem Shuntkörper gekoppelten Sensor (2104) umfasst;
wobei der Shuntkörper eine teilweise zylindrische Form mit einem Spalt (2128) aufweist, der ein erstes Ende (2126) des Shuntkörpers von einem zweiten Ende (2127) des Shuntkörpers trennt; und
wobei der Shuntkörper dazu ausgelegt ist, während des Einführens eine komprimierte Form anzunehmen, bei der das erste Ende das zweite Ende zumindest teilweise überlappt.

12. Sensorimplantatsystem, das Folgendes umfasst:
ein Shuntimplantat (1802), das einen zentralen Strömungsabschnitt umfasst, der dazu ausgelegt ist, eine Öffnung (1814) durch eine Gewebewand (1821) aufrechtzuerhalten; und
eine Sensorimplantatvorrichtung, die einen Shuntkörper (1800), der dazu ausgelegt ist, zumindest teilweise in den zentralen Strömungsabschnitt des Shuntimplantats zu passen, und einen mit dem Shuntkörper gekoppelten Sensor (1804) umfasst;
wobei der Shuntkörper mehrere Verankerungsarme (1803) umfasst, die dazu ausgelegt sind, mit dem Shuntimplantat zusammenzupassen,
wobei jeder der Verankerungsarme mit einem Halteband (1805) gekoppelt ist, das den Sensor und die Verankerungsarme miteinander verbindet; und
wobei das Halteband dazu ausgelegt ist, den Sensor zumindest teilweise über der Gewebewand auszudehnen.

13. Sensorimplantatsystem nach einem der Ansprüche 1 bis 12, wobei der Sensor dazu ausgelegt ist, sich wie folgt auszudehnen:
a) zumindest teilweise über der Öffnung durch die Gewebewand; und/oder
b) zumindest teilweise über der Gewebewand.

14. Sensorimplantatsystem nach einem der Ansprüche 1 bis 13, wobei das Shuntimplantat ferner einen oder mehrere Verankerungsarme (152, 154) umfasst.

## Revendications

1. Système d'implant de capteur, comprenant :
un implant de dérivation (1202) comprenant une partie d'écoulement centrale configurée pour maintenir une ouverture (1214) à travers une paroi tissulaire (1221) ; et
un dispositif d'implant de capteur (1200) incluant un corps de dérivation (1203) configuré pour s'adapter au moins partiellement à l'intérieur de la partie d'écoulement centrale de l'implant de dérivation et un capteur (1204) couplé au corps de dérivation ;
dans lequel le corps de dérivation comprend une boucle de fil ayant une partie proximale configurée pour prendre une première largeur à l'intérieur de la partie d'écoulement centrale de l'implant de dérivation et ayant une partie distale configurée pour s'étendre jusqu'à une seconde largeur au-delà de la partie d'écoulement centrale de l'implant de dérivation, et dans lequel la seconde largeur est supérieure à la première largeur.

2. Système d'implant de capteur selon la revendication 1, dans lequel la boucle de fil est :
a) configurée pour s'étendre jusqu'à la seconde largeur au-delà d'un premier côté (1223) de la paroi tissulaire, et dans lequel le capteur est configuré pour s'étendre au-delà d'un second côté (1222) de la paroi tissulaire ; et/ou
b) composée au moins partiellement d'un ou plusieurs alliages à mémoire de forme.

3. Système d'implant de capteur selon la revendication 1 ou la revendication 2, dans lequel la partie proximale de la boucle de fil est configurée pour être située au moins partiellement à l'intérieur de la partie d'écoulement centrale de l'implant de dérivation.

4. Système d'implant de capteur selon l'une quelconque des revendications 1 à 3, dans lequel la boucle de fil :
a) comprend une première extrémité (1226) et une seconde extrémité (1227) couplée au capteur ; et/ou
b) est configurée pour former une ou plusieurs bobines autour du capteur.

5. Système d'implant de capteur, comprenant :
un implant de dérivation (1302) comprenant une partie d'écoulement centrale configurée pour maintenir une ouverture (1314) à travers une paroi tissulaire (1321) ; et
un dispositif d'implant de capteur (1300) incluant un corps de dérivation (1303) configuré pour s'adapter au moins partiellement à l'intérieur de la partie d'écoulement centrale de l'implant de dérivation et un capteur (1304) couplé au corps de dérivation ;
dans lequel le corps de dérivation comprend une bobine ayant une partie proximale (1307) configurée pour former une première largeur à l'intérieur de la partie d'écoulement centrale de l'implant de dérivation et ayant une partie distale (1308) configurée pour former une seconde largeur au-delà de la partie d'écoulement centrale de l'implant de dérivation, et dans lequel la seconde largeur est supérieure à la première largeur.

6. Système d'implant de capteur selon la revendication 5, dans lequel le corps de dérivation est configuré pour former la seconde largeur au-delà d'un premier côté (1323) de la paroi tissulaire, et dans lequel le capteur est configuré pour s'étendre au-delà d'un second côté (1322) de la paroi tissulaire.

7. Système d'implant de capteur selon la revendication 5 ou la revendication 6, dans lequel la bobine :
a) comprend une première extrémité configurée pour former un ou plusieurs enroulements (1313) autour du capteur ;
b) comprend une seconde extrémité au niveau de la partie distale ; et/ou
c) forme plusieurs enroulements au niveau de la partie proximale et au niveau de la partie distale.

8. Système d'implant de capteur selon l'une quelconque des revendications 5 à 7, dans lequel la bobine est configurée pour maintenir le capteur :
a) au-dessus de la partie proximale de la bobine ; et/ou
b) au moins partiellement décalée par rapport à la partie proximale de la bobine.

9. Système d'implant de capteur, comprenant :
un implant de dérivation (1702) comprenant une partie d'écoulement centrale configurée pour maintenir une ouverture (1714) à travers une paroi tissulaire (1721) ; et
un dispositif d'implant de capteur incluant un corps de dérivation (1400) configuré pour s'adapter au moins partiellement à l'intérieur de la partie d'écoulement centrale de l'implant de dérivation et un capteur (1704) couplé au corps de dérivation ;
dans lequel le corps de dérivation a une forme de sablier dans laquelle une section médiane (1407) du corps de dérivation a un premier diamètre et le corps de dérivation s'étend jusqu'à un second diamètre au niveau d'une première extrémité (1426) du corps de dérivation et au niveau d'une seconde extrémité (1427) du corps de dérivation, dans lequel le second diamètre est supérieur au premier diamètre.

10. Système d'implant de capteur selon la revendication 9, dans lequel le corps de dérivation comprend :
a) un support de capteur (1405) au niveau de la première extrémité, et dans lequel le capteur est configuré pour être couplé au corps de dérivation au niveau du support de capteur ;
b) un réseau d'entretoises (1417) formant des cellules (1419), facultativement dans lequel au moins une partie des cellules sont en forme de losange ; et/ou
c) une ou plusieurs saillies (1409) au niveau de la seconde extrémité configurées pour s'accoupler avec un ou plusieurs dispositifs d'administration.

11. Système d'implant de capteur, comprenant :
un implant de dérivation (2102) comprenant une partie d'écoulement centrale configurée pour maintenir une ouverture (2114) à travers une paroi tissulaire (2121) ; et
un dispositif d'implant de capteur incluant un corps de dérivation (2100) configuré pour s'adapter au moins partiellement à l'intérieur de la partie d'écoulement centrale de l'implant de dérivation et un capteur (2104) couplé au corps de dérivation ;
dans lequel le corps de dérivation a une forme cylindrique partielle avec un espace (2128) séparant une première extrémité (2126) du corps de dérivation d'une seconde extrémité (2127) du corps de dérivation ; et
dans lequel le corps de dérivation est configuré pour prendre une forme comprimée pendant l'administration, dans lequel la première extrémité chevauche au moins partiellement la seconde extrémité.

12. Système d'implant de capteur, comprenant :
un implant de dérivation (1802) comprenant une partie d'écoulement centrale configurée pour maintenir une ouverture (1814) à travers une paroi tissulaire (1821) ; et
un dispositif d'implant de capteur incluant un corps de dérivation (1800) configuré pour s'adapter au moins partiellement à l'intérieur de la partie d'écoulement centrale de l'implant de dérivation et un capteur (1804) couplé au corps de dérivation ;
dans lequel le corps de dérivation comprend plusieurs bras d'ancrage (1803) configurés pour s'accoupler à l'implant de dérivation,
dans lequel chacun des bras d'ancrage est couplé à un filin de fixation (1805) reliant le capteur et les bras d'ancrage ; et
dans lequel le filin de fixation est configuré pour étendre le capteur au moins partiellement sur la paroi tissulaire.

13. Système d'implant de capteur selon l'une quelconque des revendications 1 à 12, dans lequel le capteur est configuré pour s'étendre :
a) au moins partiellement sur l'ouverture à travers la paroi tissulaire ; et/ou
b) au moins partiellement sur la paroi tissulaire.

14. Système d'implant de capteur selon l'une quelconque des revendications 1 à 13, dans lequel l'implant de dérivation comprend en outre un ou plusieurs bras d'ancrage (152, 154).
